# EUROPEAN PATENT APPLICATION

(11) **EP 2 330 220 A1**
(43) Date of publication of application: **08.06.2011**
(21) Application number: 09808331.4
(22) Date of filing: 18.08.2009
(51) Int. Cl.: C12Q 1/68, C12N 15/09, G01N 33/53

(54) **METHOD FOR QUANTIFICATION OR DETECTION OF DNA**

(30) Priority: 19.08.2008 JP 2008210466
(71) Applicant: Sumitomo Chemical Company, Limited, Tokyo 104-8260 (JP)
(72) Inventor: TOMIGAHARA, Yoshitaka, Toyonaka-shi Osaka 560-0013 (JP); SATOH, Hideo, Ibaraki-shi Osaka 567-0826 (JP); TARUI, Hirokazu, Toyonaka-shi Osaka 561-0802 (JP)
(74) Representative: Ford, Hazel
(86) International application number: PCT/JP2009/064688
(87) International publication number: WO 2010/021396

(57) **Abstract**

The present invention relates to a method for quantifying or detecting DNA having a target DNA region, and so on.

SEQUENCE LISTING

<110> Sumitomo Chemical Co., Ltd.

<120> Method for determining or detecting DNA

<130> S21223W001

<150> JP2008-210466
<151> 2008-08-19

<160> 17

<210> 1
<211> 332
<212> DNA
<213> Homo sapiens

<400> 1
tagaatatcc aatacagaga agtgcttaaa ggagctgatg gagctgaaaa ccaaggctcg 60
agaactacgt gaagaatgca gaagcctcag gagccgatgc gatcaactgg aagaaagggt 120
atcagcaatg gaagatgaaa tgaatgaaat gaagcgagaa gggaagttta gagaaaaaag 180
aataaaaaga aatgagcaaa gcctccaaga aatatgggac tatgtgaaaa gaccaaatct 240
acgtctgatt ggtgtacctg aaagtgatgt ggagaatgga accaagttgg aaaacactct 300
gcaggatatt atccaggaga acttccccaa tc 332

<210> 2
<211> 267
<212> DNA
<213> Homo sapiens

<400> 2
tagaactcag gattaagaat ctcactcaaa gccgctcaac tacatggaaa ctgaacaacc 60

tgctcctgaa tgactactgg gtacataacg aaatgaaggc agaaataaag atgttctttg 120
aaaccaacga gaacaaagac accacatacc agaatctctg ggacgcattc aaagcagtgt 180
gtagagggaa atttatagca ctaaatgcct acaagagaaa gcaggaaaga tccaaaattg 240
acaccctaac atcacaatta aaagaac 267

<210> 3
<211> 85
<212> DNA
<213> Homo sapiens

<400> 3
cgggcgcggt ggctcacgcc tgtaatccca gcactttggg aggccgaggt gggcggatca 60
cgaggtcagg agatcgagac catcc 85

<210> 4
<211> 20
<212> DNA
<213> Artificial Sequence

<220>
<223> Designed biotinated oligonucleotide for fixation

<400> 4
ggctcctgag gcttctgcat 20

<210> 5
<211> 48
<212> DNA
<213> Artificial Sequence

<220>
<223> Designed oligonucleotide
<220>

<221> modified_base
<222> 25,27,29,31,33,35,37,39,41,43,45,47
<223> n=m5c

<400> 5
taagcacttc tctgtattgg atatnanana nanananana nananana 48

<210> 6
<211> 21
<212> DNA
<213> Artificial Sequence

<220>
<223> Designed biotinated oligonucleotide for fixation

<400> 6
ccagtagtca ttcaggagca g 21

<210> 7
<211> 47
<212> DNA
<213> Artificial Sequence

<220>
<223> Designed oligonucleotide
<220>
<221> modified_base
<222> 24,26,28,30,32,34,36,38,40,42,44,46
<223> n=m5c

<400> 7
tgagtgagat tcttaatcct gagnananan anananana n ananana 47

<210> 8
<211> 22
<212> DNA
<213> Artificial Sequence

<220>
<223> Designed biotinated oligonucleotide for fixation

<400> 8
tcaggtacac caatcagacg ta                                                22

<210> 9
<211> 44
<212> DNA
<213> Artificial Sequence

<220>
<223> Designed oligonucleotide
<220>
<221> modified_base
<222> 21,23,25,27,29,31,33,35,37,39,41,43
<223> n=m5c

<400> 9
atcggctcct gaggcttctg nanananana nanananana nana                        44

<210> 10
<211> 21
<212> DNA
<213> Artificial Sequence

<220>
<223> Designed oligonucleotide

<400> 10

ggatggtctc gatctcctga c                                                21

<210> 11

<211> 44

<212> DNA

<213> Artificial Sequence

<220>

<223> Designed oligonucleotide

<220>

<221> modified_base

<222> 21,23,25,27,29,31,33,35,37,39,41,43

<223> n=m5c

<400> 11
   gattacaggc gtgagccacc nanananana nanananana nana                   44

<210> 12

<211> 24

<212> DNA

<213> Artificial Sequence

<220>

<223> Designed oligonucleotide

<400> 12

taagcacttc tctgtattgg atat                                             24

<210> 13

<211> 23

<212> DNA

<213> Artificial Sequence

<220>
<223> Designed oligonucleotide

<400> 13
tgagtgagat tcttaatcct gag                                                23

<210> 14
<211> 20
<212> DNA
<213> Artificial Sequence

<220>
<223> Designed oligonucleotide

<400> 14
atcggctcct gaggcttctg                                                    20

<210> 15
<211> 20
<212> DNA
<213> Artificial Sequence

<220>
<223> Designed oligonucleotide

<400> 15
gattacaggc gtgagccacc                                                    20

<210> 16
<211> 20
<212> DNA

<213> Artificial Sequence

<220>
<223> Designed oligonucleotide for amplification

<400> 16
ggtggctcac gcctgtaatc                                                    20

<210> 17
<211> 21
<212> DNA
<213> Artificial Sequence

<220>
<223> Designed oligonucleotide for amplification

<400> 17
ggatggtctc gatctcctga c                                                  21

## Description

### TECHNICAL FIELD

The present invention relates to a method for quantifying or detecting DNA comprising a target DNA region contained in a specimen, and so on.

### BACKGROUND ART

Known as a method for quantifying or detecting DNA comprising a target DNA region contained in a specimen are, for example, a method of detecting DNA amplified by a chain reaction of DNA synthesis by DNA polymerase (Polymerase Chain Reaction; hereinafter, sometimes referred to as PCR) after extraction of biological genomic DNA contained in a specimen, a method of detecting DNA by hybridization of a fluorescent-labeled oligonucleotide with a target DNA region possessed by DNA in a biological specimen, and so on (see, for example, J. Cataract. Refract. Surg., 2007;33(4):635-641, and Environ. Mol. Mutagen., 1991;18(4):259-262).

### DISCLOSURE OF THE INVENTION

It is an object of the present invention to provide a method for quantifying or detecting DNA comprising a target DNA region contained in a specimen in a simple and convenient manner.

The present invention includes the following inventions.

### [Invention 1]

A method for quantifying or detecting DNA which comprises a target DNA region existing plurally in genome and is contained in a specimen, comprising:
(1) First step of preparing a specimen containing a test oligonucleotide which is DNA comprising a target DNA region existing plurally in genome,
(2) Second step of mixing the test oligonucleotide contained in the specimen prepared in First step, a detection oligonucleotide capable of complementarily binding with the test oligonucleotide, a specific oligonucleotide capable of complementarily binding with the test oligonucleotide and a support capable of binding with the specific oligonucleotide, to form a detection complex comprising the test oligonucleotide, the detection oligonucleotide, the specific oligonucleotide and the support, and
(3) Third step of quantifying or detecting said DNA comprising the target DNA region by detecting the detection oligonucleotide contained in the detection complex formed in Second step by its identification function.

### [Invention 2]

The method according to Invention 1, wherein the detection oligonucleotide is a composite detection oligonucleotide comprising a plurality of oligonucleotides containing a plurality of methylation sites.

### [Invention 3]

The method according to Invention 1 or 2, wherein the identification function of the detection oligonucleotide is an identification function of a methylated DNA antibody that binds with methylated DNA of the detection oligonucleotide.

### [Invention 4]

The method according to any one of Inventions 1 to 3, wherein the DNA comprising the target DNA region existing plurally in genome comprises a nucleotide sequence constituting a repetition sequence in genome or a part thereof.

### [Invention 5]

The method according to any one of Inventions 1 to 3, wherein the DNA comprising the target DNA region existing plurally in genome is DNA comprising a nucleotide sequence of a duplicate gene or a pseudo gene in genome, or a part thereof.

### [Invention 6]

The method according to any one of Inventions 1 to 3, wherein the DNA comprising the target DNA region existing plurally in genome is DNA comprising a nucleotide sequence classified into a LINE or an Alu sequence.

### [Invention 7]

The method according to Invention 4, wherein the nucleotide sequence constituting the repetitive sequence in genome comprises any of the following nucleotide sequences:
(1) the nucleotide sequence of SEQ ID NO:1, or a nucleotide sequence having 80% or more sequence identity to the same;
(2) the nucleotide sequence of SEQ ID NO:2, or a nucleotide sequence having 80% or more sequence identity to the same; and
(3) the nucleotide sequence of SEQ ID NO:3, or a nucleotide sequence having 80% or more sequence identity to the same.

### [Invention 8]

The method according to any one of Inventions 1 to 7, wherein the detection oligonucleotide comprises any of the following nucleotide sequences:
(1) a partial sequence of the nucleotide sequence of SEQ ID NO:1, or a nucleotide sequence having 80% or more sequence identity to the same;
(2) a complementary sequence to a partial sequence of the nucleotide sequence of SEQ ID NO:1, or a nucleotide sequence having 80% or more sequence identity to the same;
(3) a partial sequence of the nucleotide sequence of SEQ ID NO:2, or a nucleotide sequence having 80% or more sequence identity to the same;
(4) a complementary sequence to a partial sequence of the nucleotide sequence of SEQ ID NO:2, or a nucleotide sequence having 80% or more sequence identity to the same;
(5) a partial sequence of the nucleotide sequence of SEQ ID NO:3, or a nucleotide sequence having 80% or more sequence identity to the same;
(6) a complementary sequence to a partial sequence of the nucleotide sequence of SEQ ID NO:3, or a nucleotide sequence having 80% or more sequence identity to the same;
(7) the nucleotide sequence of SEQ ID NO:4, or a nucleotide sequence having 80% or more sequence identity to the same;
(8) a complementary sequence to the nucleotide sequence of SEQ ID NO:4, or a nucleotide sequence having 80% or more sequence identity to the same;
(9) the nucleotide sequence of SEQ ID NO:6, or a nucleotide sequence having 80% or more sequence identity to the same;
(10) a complementary sequence to the nucleotide sequence of SEQ ID NO:6, or a nucleotide sequence having 80% or more sequence identity to the same;
(11) the nucleotide sequence of SEQ ID NO:8, or a nucleotide sequence having 80% or more sequence identity to the same;
(12) a complementary sequence to the nucleotide sequence of SEQ ID NO:8, or a nucleotide sequence having 80% or more sequence identity to the same;
(13) the nucleotide sequence of SEQ ID NO:10, or a nucleotide sequence having 80% or more sequence identity to the same;
(14) a complementary sequence to the nucleotide sequence of SEQ ID NO:10, or a nucleotide sequence having 80% or more sequence identity to the same;
(15) the nucleotide sequence of SEQ ID NO:12, or a nucleotide sequence having 80% or more sequence identity to the same;
(16) a complementary sequence to the nucleotide sequence of SEQ ID NO:12, or a nucleotide sequence having 80% or more sequence identity to the same;
(17) the nucleotide sequence of SEQ ID NO:13, or a nucleotide sequence having 80% or more sequence identity to the same;
(18) a complementary sequence to the nucleotide sequence of SEQ ID NO:13, or a nucleotide sequence having 80% or more sequence identity to the same;
(19) the nucleotide sequence of SEQ ID NO:14, or a nucleotide sequence having 80% or more sequence identity to the same;
(20) a complementary sequence to the nucleotide sequence of SEQ ID NO:14, or a nucleotide sequence having 80% or more sequence identity to the same;
(21) the nucleotide sequence of SEQ ID NO:15, or a nucleotide sequence having 80% or more sequence identity to the same; and
(22) a complementary sequence to the nucleotide sequence of SEQ ID NO:15, or a nucleotide sequence having 80% or more sequence identity to the same.

### [Invention 9]

The method according to any one of Inventions 1 to 7, wherein the specific oligonucleotide comprises any of the following nucleotide sequences:
(1) a partial sequence of the nucleotide sequence of SEQ ID NO:1, or a nucleotide sequence having 80% or more sequence identity to the same;
(2) a complementary sequence to a partial sequence of the nucleotide sequence of SEQ ID NO:1, or a nucleotide sequence having 80% or more sequence identity to the same;
(3) a partial sequence of the nucleotide sequence of SEQ ID NO:2, or a nucleotide sequence having 80% or more sequence identity to the same;
(4) a complementary sequence to a partial sequence of the nucleotide sequence of SEQ ID NO:2, or a nucleotide sequence having 80% or more sequence identity to the same;
(5) a partial sequence of the nucleotide sequence of SEQ ID NO:3, or a nucleotide sequence having 80% or more sequence identity to the same;
(6) a complementary sequence to a partial sequence of the nucleotide sequence of SEQ ID NO:3, or a nucleotide sequence having 80% or more sequence identity to the same;
(7) the nucleotide sequence of SEQ ID NO:4, or a nucleotide sequence having 80% or more sequence identity to the same;
(8) a complementary sequence to the nucleotide sequence of SEQ ID NO:4, or a nucleotide sequence having 80% or more sequence identity to the same;
(9) the nucleotide sequence of SEQ ID NO:6, or a nucleotide sequence having 80% or more sequence identity to the same;
(10) a complementary sequence to the nucleotide sequence of SEQ ID NO:6, or a nucleotide sequence having 80% or more sequence identity to the same;
(11) the nucleotide sequence of SEQ ID NO:8, or a nucleotide sequence having 80% or more sequence identity to the same;
(12) a complementary sequence to the nucleotide sequence of SEQ ID NO:8, or a nucleotide sequence having 80% or more sequence identity to the same;
(13) the nucleotide sequence of SEQ ID NO:10, or a nucleotide sequence having 80% or more sequence identity to the same;
(14) a complementary sequence to the nucleotide sequence of SEQ ID NO:10, or a nucleotide sequence having 80% or more sequence identity to the same;
(15) the nucleotide sequence of SEQ ID NO:12, or a nucleotide sequence having 80% or more sequence identity to the same;
(16) a complementary sequence to the nucleotide sequence of SEQ ID NO:12, or a nucleotide sequence having 80% or more sequence identity to the same;
(17) the nucleotide sequence of SEQ ID NO:13, or a nucleotide sequence having 80% or more sequence identity to the same;
(18) a complementary sequence to the nucleotide sequence of SEQ ID NO:13, or a nucleotide sequence having 80% or more sequence identity to the same;
(19) the nucleotide sequence of SEQ ID NO:14, or a nucleotide sequence having 80% or more sequence identity to the same;
(20) a complementary sequence to the nucleotide sequence of SEQ ID NO:14, or a nucleotide sequence having 80% or more sequence identity to the same;
(21) the nucleotide sequence of SEQ ID NO:15, or a nucleotide sequence having 80% or more sequence identity to the same; and
(22) a complementary sequence to the nucleotide sequence of SEQ ID NO:15, or a nucleotide sequence having 80% or more sequence identity to the same.

### [Invention 10]

The method according to any one of Inventions 1 to 9, wherein the detection oligonucleotide is a methylated oligonucleotide containing methylated cytosine.

### [Invention 11]

The method according to any one of Inventions 1 to 10, wherein the identification function of the detection oligonucleotide is detection of methylcytosine, detection by a methylated DNA antibody, or detection by a methylcytosine antibody.

### [Invention 12]

The method according to any one of Inventions 1 to 11, wherein the specimen is any of the following specimen:
(a) mammalian blood, body fluid, excreta, body secretion, cell lysate, or tissue lysate;
(b) DNA extracted from one selected from the group consisting of mammalian blood, body fluid, excreta, body secretion, cell lysate, and tissue lysate;
(c) DNA prepared by using as a template RNA extracted from one selected from the group consisting of mammalian tissue, cell, tissue lysate and cell lysate;
(e) DNA extracted from bacterium, fungus or virus; or
(f) DNA prepared by using as a template RNA extracted from bacterium, fungus or virus.

### [Invention 13]

The method according to any one of Inventions 1 to 12, wherein the DNA biological specimen comprising the target DNA region is any of the following DNAs comprising a target DNA region:
(a) DNA digested in advance with a restriction enzyme recognition cleavage site for which is not present in the target DNA region;
(b) DNA comprising a target DNA region and being purified in advance;
(c) free DNA comprising a target DNA region in blood;
(d) DNA comprising a target DNA region and being derived from microbial genome; or
(e) DNA comprising a target DNA region and having been generated from RNA by a reverse transcriptase.

### [Invention 14]

The method according to any one of Inventions 1 to 13, wherein concentration of sodium salt in a solution used in a DNA extracting operation for preparing DNA which is a specimen in First step is 100 mM or more and 1000 mM or less.

### [Invention 15]

The method according to any one of Inventions 1 to 13, wherein concentration of sodium salt in a solution used in a DNA extracting operation for preparing DNA which is a specimen in First step is 100 mM or more and 200 mM or less.

### [Invention 16]

A method for selecting a specimen from a cancer patient comprising a step of evaluating that a specimen from a test subject is a specimen from a cancer patient when there is significant difference between a quantification result or a detection result of DNA quantified or detected by using the specimen from the test subject according to the method of any one of Inventions 1 to 15 and a quantification result or a detection result of DNA quantified or detected by using a specimen from a healthy subject according to the same method, and identifying a specimen from a cancer patient based on a result of the evaluation.

### [Invention 17]

The method according to Invention 16, wherein the specimen is mammalian serum; and

### [Invention 18]

The method according to Invention 16 or 17, wherein DNA comprising a target DNA region is free DNA comprising the target DNA region in a mammalian serum.

### Brief description of drawings

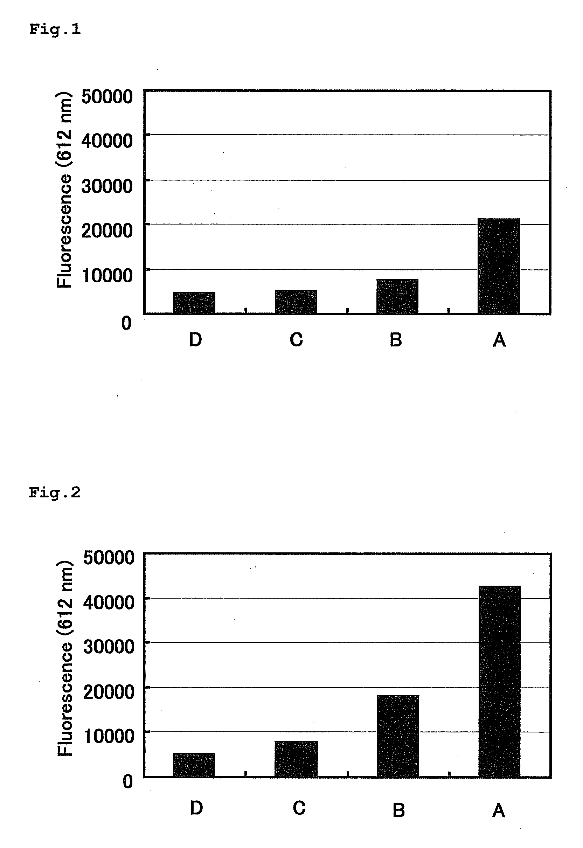

Fig. 1 is a figure showing a result of an experiment for detecting a target DNA region X in Example 1. In the figure, A represents a value of fluorescence measured for a solution having a genomic DNA concentration of 500 ng/20 µL in TE buffer using a methylated oligonucleotide M1. B represents a value of fluorescence measured for a solution having a genomic DNA concentration of 50 ng/20 µL in TE buffer using the methylated oligonucleotide M1. C represents a value of fluorescence measured for a solution having a genomic DNA concentration of 5 ng/20 µL in TE buffer using the methylated oligonucleotide M1. D represents a value of fluorescence measured for a solution having a genomic DNA concentration of 0 ng/20 µL TE buffer (negative control solution) using the methylated oligonucleotide M1.

Fig. 2 is a figure showing a result of an experiment for detecting a target DNA region Y in Example 2. In the figure, A represents a value of fluorescence measured for a solution having a genomic DNA concentration of 500 ng/20 µL in TE buffer using a methylated oligonucleotide M2. B represents a value of fluorescence measured for a solution having a genomic DNA concentration of 50 ng/20 µL in TE buffer using the methylated oligonucleotide M2. C represents a value of fluorescence measured for a solution having a genomic DNA concentration of 5 ng/20 µL in TE buffer using the methylated oligonucleotide M2. D represents a value of fluorescence measured for a solution having a genomic DNA concentration of 0 ng/20 µL TE buffer (negative control solution) using the methylated oligonucleotide M2.

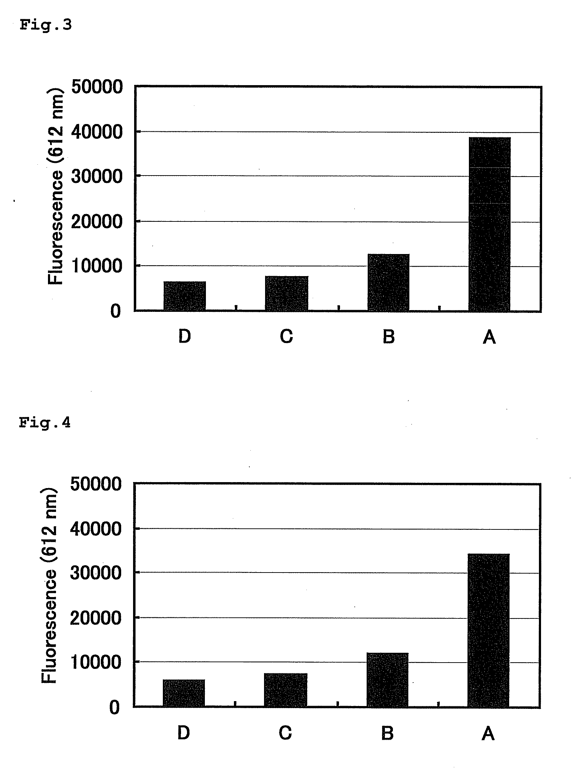

Fig. 3 is a figure showing a result of an experiment for detecting a target DNA region X in Example 3. In the figure, A represents a value of fluorescence measured for a solution having a genomic DNA concentration of 500 ng/20 µL in TE buffer using the methylated oligonucleotide M1. B represents a value of fluorescence measured for a solution having a genomic DNA concentration of 50 ng/20 µL in TE buffer using the methylated oligonucleotide M1. C represents a value of fluorescence measured for a solution having a genomic DNA concentration of 5 ng/20 µL in TE buffer using the methylated oligonucleotide M1. D represents a value of fluorescence measured for a solution having a genomic DNA concentration of 0 ng/20 µL TE buffer (negative control solution) using the methylated oligonucleotide M1.

Fig. 4 is a figure showing a result of an experiment for detecting a target DNA region X in Example 4. In the figure, A represents a value of fluorescence measured for a solution having a genomic DNA concentration of 500 ng/20 µL in TE buffer using a methylated oligonucleotide M3. B represents a value of fluorescence measured for a solution having a genomic DNA concentration of 50 ng/20 µL in TE buffer using the methylated oligonucleotide M3. C represents a value of fluorescence measured for a solution having a genomic DNA concentration of 5 ng/20 µL in TE buffer using the methylated oligonucleotide M3. D represents a value of fluorescence measured for a solution having a genomic DNA concentration of 0 ng/20 µL TE buffer (negative control solution) using the methylated oligonucleotide M3.

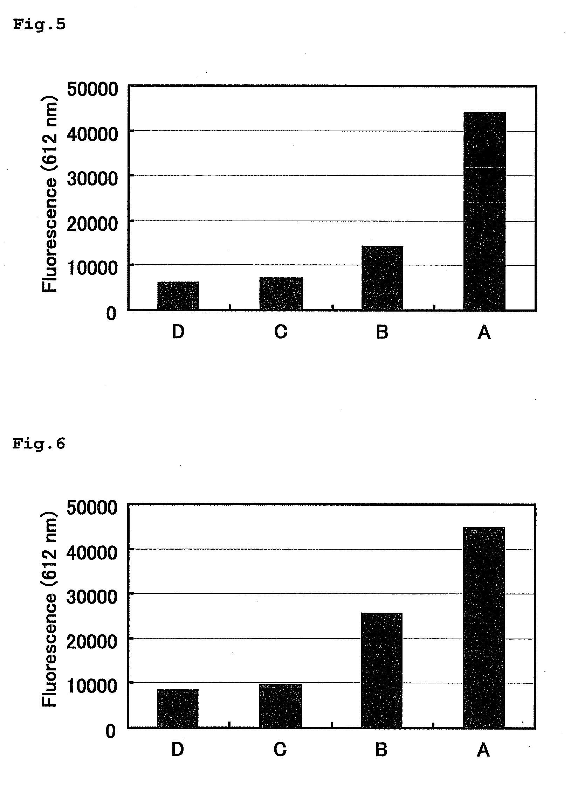

Fig. 5 is a figure showing a result of an experiment for detecting a target DNA region X in Example 5. In the figure, A represents a value of fluorescence measured for a solution having a genomic DNA concentration of 500 ng/20 µL in TE buffer using the methylated oligonucleotide M1 and the methylated oligonucleotide M3. B represents a value of fluorescence measured for a solution having a genomic DNA concentration of 50 ng/20 µL in TE buffer using the methylated oligonucleotide M1 and the methylated oligonucleotide M3. C represents a value of fluorescence measured for a solution having a genomic DNA concentration of 5 ng/20 µL in TE buffer using the methylated oligonucleotide M1 and the methylated oligonucleotide M3. D represents a value of fluorescence measured for a solution having a genomic DNA concentration of 0 ng/20 µL TE buffer (negative control solution) using the methylated oligonucleotide M1 and the methylated oligonucleotide M3.

Fig. 6 is a figure showing a result of an experiment for detecting a target DNA region Z in Example 6. In the figure, A represents a value of fluorescence measured for a solution having a genomic DNA concentration of 500 ng/20 µL in TE buffer using a methylated oligonucleotide M4. B represents a value of fluorescence measured for a solution having a genomic DNA concentration of 50 ng/20 µL in TE buffer using the methylated oligonucleotide M4. C represents a value of fluorescence measured for a solution having a genomic DNA concentration of 5 ng/20 µL in TE buffer using the methylated oligonucleotide M4. D represents a value of fluorescence measured for a solution having a genomic DNA concentration of 0 ng/20 µL TE buffer (negative control solution) using the methylated oligonucleotide M4.

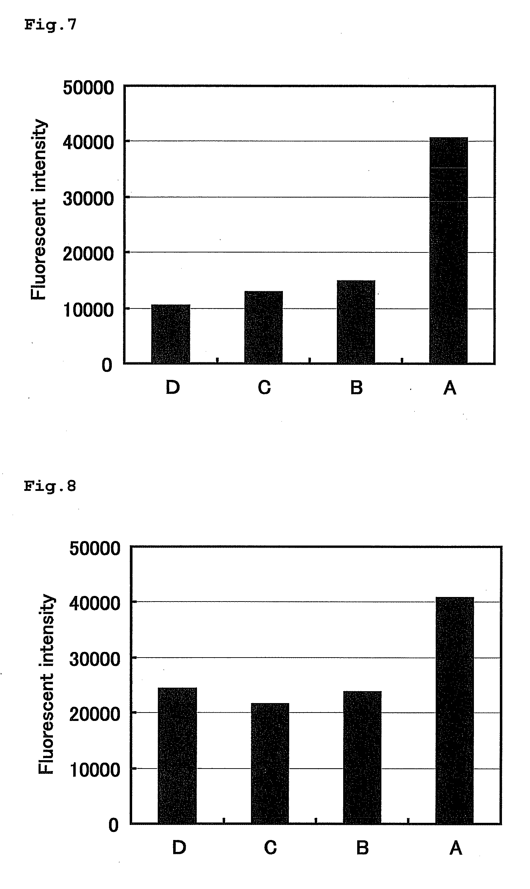

Fig. 7 is a figure showing a result of an experiment for detecting a target DNA region Z in Example 7. In the figure, Solution A represents a value of fluorescent intensity measured for a sample subjected to an operation including Treatment 1 for a serum sample A using a methylated oligonucleotide M4. Solution B represents a value of fluorescent intensity measured for a sample subjected to an operation including Treatment 1 for a serum sample B using the methylated oligonucleotide M4. Solution C represents a value of fluorescent intensity measured for a sample subjected to an operation including Treatment 1 for a serum sample C using the methylated oligonucleotide M4. D represents a value of fluorescent intensity measured for a sample subjected to an operation including Treatment 1 for a serum sample D (negative control solution) using the methylated oligonucleotide M4.

Fig. 8 is a figure showing a result of an experiment for detecting a target DNA region Z in Example 7. In the figure, Solution A represents a value of fluorescent intensity measured for a sample subjected to an operation including Treatment 2 for a serum sample A using a methylated oligonucleotide M4. Solution B represents a value of fluorescent intensity measured for a sample subjected to an operation including Treatment 2 for a serum sample B using the methylated oligonucleotide M4. Solution C represents a value of fluorescent intensity measured for a sample subjected to an operation including Treatment 2 for a serum sample C using the methylated oligonucleotide M4. D represents a value of fluorescent intensity measured for a sample subjected to an operation including Treatment 2 for a serum sample D (negative control solution) using the methylated oligonucleotide M4.

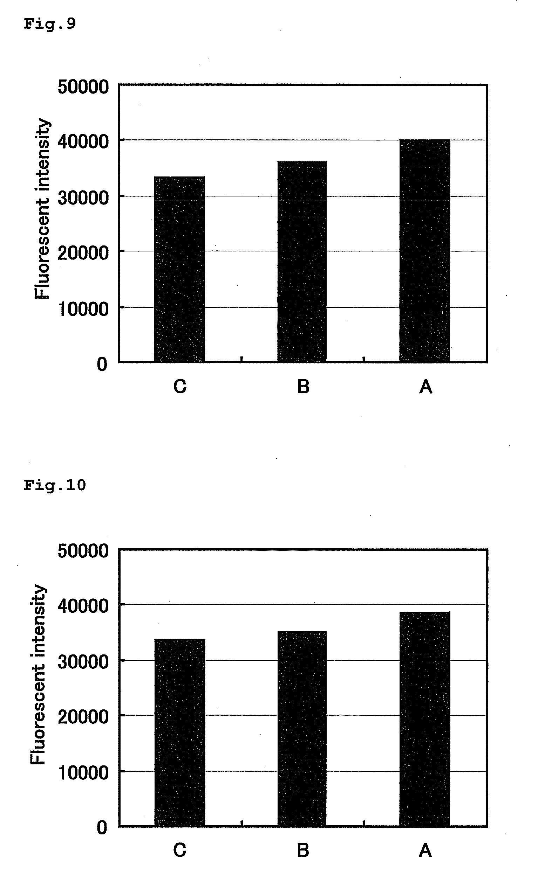

Fig. 9 is a figure showing a result of an experiment for detecting a target DNA region Z in Example 8. In the figure, Solution A represents a value of fluorescent intensity measured for a sample subjected to an operation including Treatment 1 for a serum sample A using a methylated oligonucleotide M4. Solution B represents a value of fluorescent intensity measured for a sample subjected to an operation including Treatment 1 for a serum sample B using the methylated oligonucleotide M4. Solution C represents a value of fluorescent intensity measured for a sample subjected to an operation including Treatment 1 for a serum sample C (negative control solution) using the methylated oligonucleotide M4.

Fig. 10 is a figure showing a result of an experiment for detecting a target DNA region Z in Example 8. In the figure, Solution A represents a value of fluorescent intensity measured for a sample subjected to an operation including Treatment 2 for a serum sample A using a methylated oligonucleotide M4. Solution B represents a value of fluorescent intensity measured for a sample subjected to an operation including Treatment 2 for a serum sample B using the methylated oligonucleotide M4. Solution C represents a value of fluorescent intensity measured for a sample subjected to an operation including Treatment 2 for a serum sample C (negative control solution) using the methylated oligonucleotide M4.

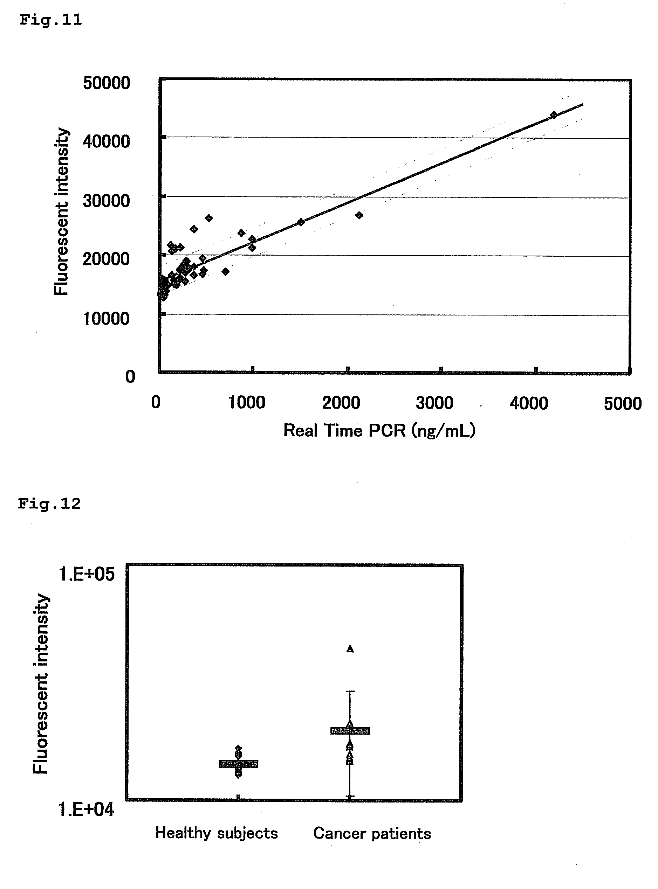

Fig. 11 is a figure showing a result of an experiment for detecting a target DNA region Z contained in a human serum in Example 9. In the figure, compared are a result detected by using a methylcytosine antibody, and the methylated oligonucleotide M4 and a 5'-end biotin-labeled oligonucleotide B4, and a result quantified by real time PCR. The graph was plotted with a detection result as the vertical axis and a quantification result by real time PCR as the horizontal axis. The straight lines in the graph represent a regression line (thick line) and a standard error range (thin line).

Fig. 12 is a figure showing a result of an experiment for detecting a target DNA region Z contained in human serum in Example 9. In the figure, for serum samples of human beings at age 59 or younger, DNA was detected using the methylcytosine antibody and the methylated oligonucleotide M4 and the 5'-end biotin-labeled-oligonucleotide B4, and plotted separately for cancer patients and healthy subjects, together with respective average values and standard deviations.

### Mode for carrying out the invention

The present method is a method of quantifying or detecting DNA comprising a target DNA region existing plurally in genome contained in a specimen, having First step of preparing a specimen containing a test oligonucleotide which is DNA comprising a target DNA region existing plurally in genome, (2) Second step of mixing the test oligonucleotide contained in the specimen prepared in First step, a detection oligonucleotide capable of complementarily binding with the test oligonucleotide, a specific oligonucleotide capable of complementarily binding with the test oligonucleotide and a support capable of binding with the specific oligonucleotide, to form a detection complex comprising the test oligonucleotide, the detection oligonucleotide, the specific oligonucleotide and the support, and (3) Third step of quantifying or detecting said DNA comprising a target DNA region by detecting the detection oligonucleotide contained in the detection complex formed in Second step by its identification function.

As the "specimen" in the present method, for example, surface adhered matters from foods, rivers, soils or general commercial products can be recited, and these specimens may contain contaminated microorganisms such as fungi, bacteria, and viruses or nucleic acids.

In foods, it is important to inspect whether there is a food poisoning bacterium and to identify a causative bacterium, and an immunological method using an antigen of the microbial surface is usually used. However, the immunological method requires a huge amount of labor to prepare an antigen, and further requires identification of a pathogenic bacterium.

In other words, since an immunological method in a microbial inspection utilizes specificity of a microbial species, not only it is difficult to examine presence or absence of a plural kinds of bacteria in one inspection, but also a huge amount of labor is required for inspection such that a PCR method or the like is used for a microorganism for which an immunological method cannot be established. The present method is able to establish an inspection method from gene even when inspection by an immunological method is difficult, and in turn able to provide an inspection method capable of concurrently detecting a plurality of microorganisms. In other words, the present method can be used for inspection of fungi, microorganisms, virus and the like existing in a non-biological specimen. Also by using the present method, it becomes possible to detect a contaminated microorganism or virus, for example, in food, and application in an examination of infection, a food contamination inspection or the like is expected.

As the specimen, (a) blood, a body fluid, excreta, a body secretion, a cell lysate or a tissue lysate derived from a mammal, (b) DNA extracted from one selected from the group consisting of blood, a body fluid, excreta, a body secretion, a cell lysate and tissue lysate derived from a mammal, (c) DNA prepared from RNA extracted from the one selected from the group consisting of a tissue, a cell, a tissue lysate and a cell lysate derived from a mammal as a template, (d) DNA extracted from a bacterium, a fungus or a virus, (e) DNA prepared from RNA extracted from a bacterium, a fungus or a virus as a template, and the like are recited. The term tissue is used in a broad sense including blood, lymph node and the like, and as the body fluid, plasma, serum, lymph and the like are recited, and as the body secretion, urine, milk and the like are recited.

As the DNA contained in the specimen, genomic DNA obtained by extraction from said biological sample or said contaminated microorganism, a DNA fragment derived from genomic DNA, or RNA can be recited. When the specimen derived from a mammal is human blood, a body fluid, a body secretion or the like, a sample collected in a clinical examination in a regular health examination of human or the like may be used. When blood is used as a specimen, by preparing plasma or serum according to a usual method from blood, and analyzing free DNA (containing DNA derived from a cancer cell such as a gastric cancer cell) contained in the prepared plasma or serum as a specimen, DNA derived from a cancer cell such as a gastric cancer cell can be analyzed avoiding DNA derived from blood cells and sensitivity of detecting a cancer cell such as a gastric cancer cell, a tissue containing the same and the like can be improved.

For obtaining genomic DNA from a specimen derived from a mammal, for example, a commercially available DNA extracting kit and the like may be used. For obtaining DNA from RNA, a kit for synthesizing DNA from RNA using a reverse transcriptase such as a commercially available cDNA preparation kit may be used. In the present method, the specimen may be DNA that is artificially synthesized.

The term "mammal" in the present method means animals classified into animal kingdom, Chordata, Chordate subphylum, Mammalia, and concrete examples include human being, monkey, marmoset, guinea pig, rat, mouse, bovine, sheep, dog, cat and the like.

The term "body fluid" in the present invention means a liquid existing between cells constituting an individual body, and concretely, plasma and interstitial fluid are recited, and it often functions to maintain homeostasis of an individual body. More concretely, lymph, tissue fluids (intercellular fluid, intercellular fluid, interstitial fluid), celomic fluid, serous cavity fluid, pleural effusion, ascetic fluid, pericardial fluid, cerebral fluid (spinal fluid), joint fluid (spinal fluid), eye aqueous fluid (aqueous fluid), cerebrospinal fluid, and the like are recited.

The term "body secretion" in the present invention is a secretion from an exocrine gland. Concrete examples include saliva, gastric juice, bile, pancreatic juice, intestinal juice, sweat, tear, runny nose, semen, vaginal lubricant, amniotic fluid, milk, and the like.

The "cell lysate" in the present invention means a lysate containing an intracellular fluid obtained, for example, by breaking cells cultured in a 10 cm plate for cell culture, namely, cell strains or primary cultured cells, blood cells and the like. As a method of breaking cell membranes, a method based on sonication, a method using a surfactant, a method using an alkaline solution and the like are recited. For lysing cells, a variety of kits and the like may be used.

Concretely, for example, after culturing cells to be confluent in a 10 cm plate, the culture solution is removed, and 0.6 mL of a RIPA buffer (1x TBS, 1% nonidet P-40, 0.5% sodium deoxysholate, 0.1% SDS, 0.004% sodium azide) is added to the plate. After shaking slowly the plate at 4°C for 15 minutes, cells adhered on the 10 cm plate are removed by using a scraper or the like, and the lysate liquid on the plate is transferred to a microtube. After adding 10 mg/mL PMSF in an amount of 1/10 volume of the lysate liquid, the tube is left still on ice for 30 to 60 minutes. Centrifugation at 10,000xg is conducted at 4°C for 10 minutes, to obtain the supernatant as a cell lysate.

The "tissue lysate" in the present invention means a lysate containing an intracellular fluid obtained by breaking cells in tissues collected from an animal such as a mammal.

More concretely, after measuring weight of the tissue obtained from an animal, the tissue is cut into small pieces with the use of a razor or the like. When a frozen tissue is sliced, it is necessary to make a smaller piece. After cutting, an ice-cooled RIPA buffer (protease inhibitor, phosphatase inhibitor and the like may be added, and for example, 10 mg/mL PMSF in an amount of 1/10 volume of the RIPA buffer may be added) is added in a rate of 3 mL per 1 g of tissue, and homogenized at 4°C. For homogenization, a sonicator or a pressurized cell grinder is used. In an operation of homogenization, the solution is constantly kept at 4°C for preventing heat generation. The homogenized liquid is transferred to a microtube, and centrifuged at 4°C for 10 minutes at 10,000xg, and the supernatant is obtained as a tissue lysate.

The "target DNA region" (hereinafter, sometimes referred to as a target region) in the present method means a DNA region intended to be detected or quantified by the present method in DNA contained in a specimen. When the specimen is DNA, the target DNA region is a predetermined nucleotide sequence on a nucleotide sequence of the DNA, and when the specimen is RNA, it is a nucleotide sequence on DNA prepared from RNA by a reverse transcriptase, and is a complementary nucleotide sequence of a predetermined nucleotide sequence intended to be detected or quantified on the RNA.

First step is a step of preparing a specimen containing a test oligonucleotide which is DNA comprising a target DNA region existing plurally in genome.

The "test oligonucleotide" in the present method means an oligonucleotide containing a target DNA region existing plurally in genome.

As the "target DNA region existing plurally in genome", any nucleotide sequence that is found plurally in genome (hereinafter, sometimes referred to as a repetitive sequence) is recited, and a nucleotide sequence that is indicative of a disease is more preferred. For example, in cancer, it is known that free DNA derived from genomic DNA in blood increases, and in this case, as the test oligonucleotide, oligonucleotides comprising a repetitive sequence in free DNA in blood and its partial sequence are recited. The quantified value of such an oligonucleotide comprising a repetitive sequence or its partial sequence can be regarded as an index representing the degree of progression of the cancer. As will be described later, the repetitive sequence may be a simple repetitive sequence (called a tandem repetitive sequence or a tandem repeat), an interspersed repetitive sequence, a duplicate gene, a pseudo gene and the like.

For obtaining genomic DNA containing a nucleotide sequence of a target DNA region, for example, a commercially available DNA extraction kit (Genfind v2 Kit (available from BECKMAN COULTER), FastPure DNA Kit (available from TAKARA BIO INC.)) and the like may be used, when the specimen is derived from a mammal.

When the specimen is a microorganism such as fungus, a general preparation method of yeast genome or the like as described in Methods in Yeast Genetics (Cold Spring Harbor Laboratory Press) may be used, while when the specimen is a prokaryote such as Escherichia coli, a general preparation method of microorganism genome or the like as described in Molecular Cloning -A Laboratory Manual- (Cold Spring Harbor Laboratory Press) may be used.

When the specimen is food, DNA may be prepared after separating a microorganism or the like from the food, and genomic DNA derived from other organism than microorganisms such as virus, and genomic DNA derived from a microorganism contained in the food may be obtained concurrently.

When the specimen is a tissue derived from a mammal, and the target DNA region is DNA derived from a virus, RNA may be extracted from the tissue using such as a commercially available RNA extraction kit (ISOGEN(311-02501)(available from NIPPON GENE CO., LTD.), or FastRNA Pro Green Kit (available from Funakoshi Corporation), FastRNA Pro Blue Kit (available from Funakoshi Corporation), FastRNA Pro Red Kit (available from Funakoshi Corporation), and the like), and DNA may be obtained by a reverse transcriptase. When the specimen is a specimen derived from a mammal, viral DNA may be extracted after extracting virus particles, or after extracting virus particles, viral RNA may be extracted using a commercially available kit (QuickGene RNA tissue kit SII, available FUJIFILM Corporation) or the like, and DNA derived from the virus may be obtained by a reverse transcriptase. RNA may be extracted from a tissue infected by a virus, and DNA derived from the virus may be obtained by a reverse transcriptase, or DNA may be obtained from a tissue infected by a virus, and DNA derived from the virus may be obtained. When DNA is obtained from RNA by a reverse transcriptase, a commercially available kit (Transcripter high fidelity cDNA synthesis kit, available from Roche Diagnostics K.K.) and the like may be used.

When the specimen is DNA prepared by using RNA from a tissue or a cell strain derived from a mammal, as a template, DNA may be obtained by a reverse transcriptase, using RNA extracted by using a commercially available RNA extraction kit from the tissue, cell strain or the like.

The "DNA comprising a target DNA region" in the present method may be DNA that is digested in advance with a restriction enzyme whose recognition cleavage site does not exist in the nucleotide sequence within the target DNA region possessed by the DNA, or may be a DNA sample that is purified in advance. Alternatively, the DNA obtained in First step may be free DNA in blood, DNA derived from microbial genome, DNA synthesized from RNA in the specimen by a reverse transcriptase and the like.

As the "target DNA region", when a DNA region synthesized from RNA by a reverse transcriptase is used, DNA synthesized from ribosomal RNA, messenger RNA, transfer RNA, and micro RNA and the like is recited as the "DNA comprising a target DNA region". As the RNA, not only the one that is transferred from genome of a host by RNA polymerase, but also the one containing virus genome whose genome is RNA are included, and any RNA is applicable.

Examples of the "DNA comprising a target DNA region" in the present method include DNA derived from microorganisms such as gram-positive bacteria, gram-negative bacteria, fungi, viruses and pathogenic protozoans, and DNA obtained from RNA derived from such microorganisms by a reverse transcriptase. For example, genomic DNA or DNA prepared by a reverse transcriptase from RNA of Mycoplasma genitalium, Mycoplasma pneumoniae, Borrelia burgdorferi B31, Rickettsia prowazekii, Treponema pallidum, Chlamydia pneumoniae, Chlamydia trachomatis, Helicobacter pylori J99, Helicobacter pylori 26695, Haemophilus influenzae Rd, Mycobacterium tuberculosis H37Rv, Pseudomonas aeruginosa, Legionella pneumophila, Serratia marcescens, Escherichia coli, Listeria monocytogenes, Salmonella enterica, Campylobacter jejuni subsp. Jejuni, Staphylococcus aureus, Vibrio parahaemolyticus, Bacillusu cereus, Clostridium botulinum, Clostridium perfringens, Yersinia enterocolitica, Yersinia pseudotuberuculosis, Trichophyton ruburum, Trichophyton mentagrophytes, Candida albicans, Cryptococcus neoformans, Aspergillus fumigatus, Pneumocystis carinii, Coccidioides immitis, Cytomegalovirus, human herpesvirus 5, Epstein-Barr virus, Human Immunodeficiency Virus, Human Papilloma Virus, Enterovirus, Norovirus Influenza Virus, Toxoplasma gondii, Cryptosporidium parvum, or Entamoeba histolytica may be used for detection of a microorganism responsible for an infection in a specimen, or a microorganism responsible for a food poisoning in food.

As the nucleotide sequence to be detected, a CRISPR (Clustered regularly interspaced short palindromic repeats) region which is a nucleotide sequence found plurally in genome and the like are recited.

Identification and detection of a microorganism using the present method can be applied for diagnosis of infection, identification of a food poisoning bacterium, identification of the kind and the number of microorganisms existing in soil or in sediment of river or lake (microbial survey in environment), identification of industrially useful microorganisms such as a microorganism producing a useful compound or a microorganism usable for food fermentation, and examination of a bacterial layer (microbial layer) in the condition that microorganisms are industrially used, for example, in a sewage treatment or in food fermentation.

The "repetitive sequence" in the present method means a nucleotide sequence for which the identical predetermined sequence is plurally found in genome. As such a repetitive sequence, a simple repetitive sequence (called a tandem repetitive sequence or a tandem repeat), an interspersed repetitive sequence and the like are known.

The simple repetitive sequence is characterized in that the identical sequences neighbor in the same orientation, and a series of nucleotide sequences such as satellite DNA, minisatellite, microsatellite, centromere, telomere, kinetochore, and ribosome group genes are known.

The interspersed repetitive sequence is characterized in that the identical sequences are interspersed without neighboring each other, and is believed to be DNA derived from retrotransposon. Interspersed repetitive sequences are classified into SINE (Short Interspersed Repetitive Element: short chain interspersed repetitive sequence) and LINE (Long Interspersed Elements:long chain interspersed repetitive sequence) depending on the length of the nucleotide sequence, and Alu sequence and LINE-1 sequence are respectively known as representative repetitive sequences as human nucleotide sequences. Also an inactive processed pseudo gene that is counter-transferred from RNA or protein, and a gene sequence amplified by gene duplication are known.

The term duplicate gene indicates the case that a plurality of genes having high homology exist on one genome, and in many cases, it includes nucleotide sequences existing in tandem near one gene. Some pseudo genes are known to be included in duplicate genes.

As a nucleotide sequence that is found plurally in genome, repetition of a relatively short nucleotide sequence is recited. Concretely, (A)n, (T)n, (GA)n, (CA)n, (TAA)n, (GGA)n, (CAGC)n, (CATA)n, (GAAA)n, (TATG)n, (TTTG)n, (TTTA)n, (TTTC)n, (TAAA)n, (TTCA)n, (TATAA)n, (TCTCC)n, (TTTCC) n (TTTAA)n, (TTTTC)n, (TTTTA)n, (TTTTG)n, (CAAAA)n, (CACCC)n, (TATATG)n, (CATATA)n, (TCTCTG)n, (AGGGGG)n, (CCCCCA)n, (TGGGGG)n (n means the number of repetition) and the like sequences are recited. Next, a sequence derived from a transcription factor is recited. Concretely, as a hAT group, MER1-Charlie and Zaphod are known, as a Tc-1 group, MER2-Tigger, Tc-1 and Mariner are known. As others, Tigger1, Tigger2a, Tigger5, Charlie4a, Charlie7 and the like are known. These "relatively short nucleotide sequence" and "sequence derived from a transcription factor" are applicable to the present method if a specific adhesion sequence and a detecting adhesion sequence as will be described later can be set. Also, satellite DNA, minisatellite, microsatellite and the like are repetitive sequences classified into simple repetitive sequences, and are applicable to the present method if a specific adhesion sequence and a detecting adhesion sequence as will be described later can be set. As a sequence having multi-copies in gene, ALR6 as a sequence existing in centromere, and U2 and U6 as snRNA are known, and also it is known that in the nucleotide sequences having a biological function such as tRNA and rRNA, there are nucleotide sequences having multi-copies in genome. As such a gene, a duplicate gene which is a gene having multi-copies in genome as a result of gene duplication can be recited.

The term duplicate gene means a gene or a gene fragment that is generated by doubling of a specific gene or gene fragment in genome due to gene duplication. Gene duplication is a phenomenon that a region of DNA including a gene is overlapped. As a cause of gene duplication, abnormality of gene recombination, translocation of retrotransposon, duplication of the entire chromosome and the like are recited. For example, when one gene is copied and inserted into genomic DNA, it is inserted into a different chromosome site in one case, and inserted near the original gene in the other case. The site where the copied gene stands in line as a result of insertion near the original gene is called a tandem repeat, and a group of genes generated by gene duplication is called a gene family.

A pseudogene means a gene having a characteristic nucleotide sequence that is assumable to have encoded a gene product (particularly protein) in a sequence of DNA, but currently loosing the function. It is assumed that it is generated as a result of mutation of the original functioning sequence. For example, there is the case where a stop codon arises by mutation and a peptide chain of a protein is shortened, so that the function as a protein is no longer effective, and there is the case where a function of a regulatory sequence required for normal transcription is impaired due to mutation such as single nucleotide substitution. In many pseudogenes, the original normal genes are remained separately, however, those becoming pseudogenes by themselves are also known. Pseudogenes are classified into three types according to the characteristic of the gene sequence. There are known the case where DNA prepared from mRNA by a reverse transcriptase of retrotransposon is inserted into genome (processed pseudogene), the case where an original gene sequence is duplicated in genome, and a part of the copies looses the function due to mutation or the like to become a pseudogene (duplicated pseudogene or non-processed pseudogene), and the case where gene in genome (in the condition of single gene with no duplicated gene) looses the function to become a pseudogene.

Currently, among the genes known as pseudogenes, transcribed examples, examples having a gene function (whether it is called a pseudogene is not determined) and the like also have been known, so that the term "pseudogene" in the present method means the "processed pseudogene" or "duplicated pseudogene (non-processed pseudogene)" rather than presence or absence of gene function or whether it is transcribed or not.

As the "nucleotide sequence found plurally in genome" which is the "repetitive sequence" in the present method, endogenous sequences considered to be derived from virus such as retrovirus, retrotransposon having a LTR (Long terminal repeat) in its end, and MaLRs (Mammalian apparent LTR-Retrotransposons), LTR derived from retrovirus and the like can be recited.

For example, as the LTR derived from a retrovirus, concretely, subfamilies such as LTR1, LTR1B, LTR5, LTR7, LTR8, LTR16A1, LTR16A1, LTR16C, LTR26, LTR26E, MER48, and MLT2CB are known. The LTRs derived from a retrotransposon are classified into classes of ERV, ERVK and ERVL, and concrete examples include subfamilies such as LTR8A, LTR28, MER21B, MER83, MER31B, MER49, MER66B, HERVH, ERVL, LTR16A1, LTR33A, LTR50, LTR52, MLT2A1, MLT2E, MER11C, and MER11C. Further, MaLRs indicate DNA factors including LTRs in both ends likewise a typical retrotransposon, wherein an internal sequence sandwiched between LTRs is not derived from a retrovirus. For example, subfamilies such as MLT1A1, MLT1A2, MLT1B, MLT1C, MLT1D, MLT1F, MLT1G, MLT1H, MLT1J, MLT1K, MLT1I, MLT2CB, MSTA, MSTA-int, MSTB, THE1A, THE1B, THE1B-internal, and THE1 can be recited.

The interspersed repetitive sequences are characterized by being interspersed without neighboring each other, and are considered to be derived from a retrotransposon. Further, the interspersed repetitive sequences are classified into SINE (Short Interspersed Repetitive Element: short chain interspersed repetitive sequences) and LINE (Long Interspersed Elements: long-chain interspersed repetitive sequences) according to the length. Most of SINEs are sequences belonging to the Alu family. A common feature is that it has a sequence of 3'-side or a sequence of 5'-side of 7SL RNA, and that it has an AT-Rich region sandwiched between a Left-monomer and a Right-monomer. As subfamilies of the Alu family, Alu, AluJb, AluJo, AluSc, AluSg, AluSp, AluSq, AluSx, AluY, and FAM (Fossil Alu Monomer), FLAM (Free Left Alu Monomer) having a sequence of FAM, and FRAM (Free Right Alu Monomer) can be recited. As SINEs other than the Alu family, MIR, and Ther/MIR3 are known, and MIR and MIR3 are known as respective subfamilies. As subfamilies of the Alu family including other biological species, B1, B2, B4, PB1, PB1D and so on are known. As LINEs, subfamilies of LINE1 to Line23 are reported, and it is known that subfamilies such as LINE-1, LINE2, and LINE3 broadly exist in a genome. As for LINE-1, for example, L1M1, L1M2, L1M3, L1M3d, L1M4, L1M4c, L1MA2, L1MA7, L1MA8, L1MA9, L1MB1, L1MB1, L1MB3, L1MB4, L1MB5, L1MB6, L1MB7, LIMCa, L1MCb, L1MC2, L1MC3, L1MC4, LlMC4a, L1MC5, L1MDa, L1ME, L1MEc, L1MEd, L1MEg, L1ME1, L1ME2, L1ME3, L1ME3A, L1ME3B, LlME4a, L1PB3, L1P4, L1PA2, L1PA3, L1PA4, L1PA5, L1PA6, L1PA7, L1PA10, L1PA12, L1PA13, L1PA14, LlPA16, L1PB1, L1PB3, L1PB4, L1PREC2, and HAL1 are known, and as LINE-2, subfamilies such as L2 and L2c are known. For example, if the later-described specific adhesion sequence and the detection adhesion sequence can be set, for a sequence common to the Alu family or subfamilies of Alu, or the LINE-1 family or subfamilies of LINE-1, a plurality of detection objects can be set in one genome, so that sensitivity of genome detection can be improved.

As a target DNA region, concretely, for example, a partial sequence of LINE-1 (the nucleotide sequence of SEQ ID NO:1 or SEQ ID NO:2), a partial sequence of Alu (the nucleotide sequence of SEQ ID NO:3) or nucleotide sequences having homology to these sequences can be recited.

For example, when a repetitive sequence in a certain region needs to be examined, databases such as Repbase (http://www.girinst.org/repbase/) and RepeatMasker (http://www.repeatmasker.org/) may be used because it is difficult to retrieve a general sequence retrieving database such as PuMed. Measuring these repetitive sequences can be treated, for example, as a surrogate marker of a free DNA amount in blood, and can be utilized for identification of an organism species when an organism species-specific repetitive sequence is noted.

Examples of the repetitive sequence in genome include those comprising nucleotide sequences specifically shown below:
(1) the nucleotide sequence of SEQ ID NO:1, or a nucleotide sequence having 80% or more sequence identity to the same;
(2) the nucleotide sequence of SEQ ID NO:4, or a nucleotide sequence having 80% or more sequence identity to the same; and
(3) the nucleotide sequence of SEQ ID NO:9, or a nucleotide sequence having 80% or more sequence identity to the same.

Second step is a step of mixing the test oligonucleotide contained in the specimen prepared in First step, a detection oligonucleotide capable of complementarily binding with the test oligonucleotide, a specific oligonucleotide capable of complementarily binding with the test oligonucleotide, and a support capable of binding with the specific oligonucleotide, to form a detection complex comprising the test oligonucleotide, the detection oligonucleotide, the specific oligonucleotide and the support.

The "detection oligonucleotide" in Second step means an oligonucleotide capable of complementarily binding with the test oligonucleotide wherein a base of nucleotide constituting the oligonucleotide has an identification function for detecting or quantifying the detection oligonucleotide, and comprising a detecting adhesion sequence which is a nucleotide sequence for binding with the DNA comprising a target DNA region (test oligonucleotide) by complementation, and a detection sequence linked with the detecting adhesion sequence. The "detection oligonucleotide" may comprise a nucleotide sequence capable of complementarily binding with a repetitive sequence in human genome, a nucleotide sequence of a duplicate gene or a pseudo gene as will be described later or a part of the same. The "detection oligonucleotide" in Second step in the present method may be in the form of a complex detection oligonucleotide as will be described later. Also the detection oligonucleotide may be an oligonucleotide containing a plurality of methylated DNA, or may be a methylated oligonucleotide containing methylated cytosine.

The "detecting adhesion sequence" in the detection oligonucleotide is an oligonucleotide comprising a part of a nucleotide sequence that is complementary to a nucleotide sequence comprising a target DNA region, and is a sequence having a homology of 75% or more, and preferably 90% or more with respect to the nucleotide sequence part of the DNA comprising a target DNA region with which the detecting adhesion sequence is capable of pairing. The detecting adhesion sequence has only to be designed to have a nucleotide sequence binding with a target DNA region or with the vicinity of the target DNA region in the test oligonucleotide, and is capable of forming a detection complex as will be described later in Second step.

Here, the detecting adhesion sequence may be any nucleotide sequence as far as a detection complex comprising the detection oligonucleotide, the test oligonucleotide, the specific oligonucleotide as will be described later and the support that are bound each other can be formed, and the one that will not inhibit binding between the specific oligonucleotide as will be described later and the test oligonucleotide is particularly preferred. In an identical repetitive sequence (in target DNA region), only one detecting adhesion sequence may be designed or two or more detecting adhesion sequences may be designed. When two or more detecting adhesion sequences are designed, the plurality of detecting adhesion sequences and the later-described specific adhesion sequence will preferably not mutually inhibit binding with the DNA comprising a target DNA region. The length of the nucleotide sequence of the detecting adhesion sequence is 5 bp to 50 bp, and preferably 10 bp to 30 bp.

The "detection sequence" has only to be a nucleotide sequence that can be used while it is bound to the foregoing detecting adhesion sequence, and has an identification function. In other words, it may be an oligonucleotide formed of a characteristic nucleotide sequence for detection or quantification, or may be a molecule itself having an identification function, or may be an oligonucleotide to which a molecule having an identification function is bound. For example, it may be FITC, a radioactive label, or an oligonucleotide labeled with FITC or a radioisotope.

Also, for example, the detection sequence may be methylated DNA. Concretely, when the methylated oligonucleotide is 5-methylcytosine, the detection sequence can be detected or quantified by allowing a methylcytosine antibody having an identification function to bind. The detection sequence may be a nucleotide sequence capable of complementarily binding with the methylated oligonucleotide.

The term "molecule capable of combining an identification function" means a molecule other than an oligonucleotide that is bound to the oligonucleotide for conferring the identification function to the detection oligonucleotide. For example, when the identification function is horseradish Peroxidase (HRP) with which a FITC antibody is labeled as will be described later, FITC is a "molecule capable of combining an identification function" when the FITC is bound to the detection oligonucleotide.

The "identification function" is a function capable of detecting or quantifying a detection oligonucleotide. The identification function may be any function possessed by the detection oligonucleotide, and for example, an identification function based on labeling of the detection oligonucleotide, and an identification function imparted to the detection oligonucleotide by a detection molecule binding to the detection oligonucleotide can be recited. Concretely, characteristics of fluorescence or coloring of a detection oligonucleotide labeled at its 5'-end or 3'-end with europium, gold colloid, latex bead, radioactive isotope, a fluorescent substance (such as FITC), horseradish Peroxidase (HRP), alkaline phosphatase or the like can be recited.

For detection of europium, after adding and mixing Enhancement Solution (available from PerkinElmer, Inc.), and keeping still for about 45 minutes at room temperature, fluorescence (excitation 340nm/fluorescence 612 nm) may be measured by a fluorescent detector. When the detection oligonucleotide is a methylated oligonucleotide, as a detection molecule, concretely, a methylated DNA antibody, an osmium complex (J. Am. Chem. Soc.,2007;129:5612-5620) and the like can be recited. Here, the methylated oligonucleotide is an oligonucleotide wherein at least one of bases of nucleotides constituting the oligonucleotide is methylated, and concrete examples include oligonucleotides including 5-methylcytosine, 6-methyladenine and the like. Further, when the detection oligonucleotide is labeled with FITC, a FITC antibody can be recited as a detection molecule.

The "detection molecule" has only to have a property of detecting or quantifying a detection oligonucleotide. The detection molecule may recognize a detection sequence of a detection oligonucleotide, or may be bound in advance to a detection oligonucleotide. The detection molecule has only to have a property of specifically binding with a detection oligonucleotide, and having an "identification function" which is a function or characteristic utilized for quantification or detection, or capable of being provided with "identification function". Concretely, the detection molecule has only to be capable of binding with the methylated oligonucleotide to detect the methylated oligonucleotide, and specifically binding with the methylated oligonucleotide to exhibit the identification function when the detection sequence is a methylated oligonucleotide. As others, for example, the detection molecule may be a methylated DNA antibody. When the detection sequence is a detection molecule itself, it is not necessary to add a new detection molecule for detecting the detection oligonucleotide, and by detecting the detection molecule incorporated into the detection oligonucleotide, it becomes possible to detect the detection oligonucleotide.

When the detection molecule is a methylated DNA antibody, it may be utilized as an identification function used for quantification or detection in the following manner. Concretely, labels such as europium label, gold colloid label, latex bead label, radioisotope label, fluorescent substance (e.g., FITC) label, horseradish Peroxidase (HRP) label, alkaline phosphatase label, biotin label and the like are functions using fluorescence, coloring and the like. As a method of imparting an identification function to the antibody functioning as a detection molecule, an identification function may be directly bound to the antibody which is a detection molecule, or a secondary antibody or a tertiary antibody having an identification function may be bound to the antibody which is a detection molecule. Concretely, since an antibody labeled with a fluorescent substance, an antibody labeled with horseradish Peroxidase (HRP), an antibody labeled with alkaline phosphatase, an antibody labeled with biotin, and an antibody labeled with europium can be used as a secondary antibody or a tertiary antibody. Also an antibody to which a substrate detectable by an enzyme cycle method is bound may be used. As a quantifying or detecting means of such an identification function, for example, measurement by a radiation detector, a spectrophotometer or the like, or visual check can be recited. For example, as a case of detecting or quantifying a detection oligonucleotide according to its identification function, when a secondary antibody to which europium is added is concretely used, Enhancement Solution (available from PerkinElmer, Inc.) is added and mixed after allowing the secondary antibody to bind with the later-described detection complex, and left still for about 45 minutes at room temperature. Thereafter, fluorescence (excitation 340 nm/fluorescence 612 nm) may be measured by a fluorescent detector.

When a methylated DNA antibody is allowed to bind with methylated DNA on the detection oligonucleotide and detection or quantification is made according to its function, concretely, after allowing the methylated DNA antibody to bind with the detection complex, a secondary antibody against the methylated DNA antibody (for example, Eu-N1-labeled mouse IgG antibody:available from PerkinElmer, Inc.) is added, and left still for about 1 hour at room temperature, to thereby prompt binding of the secondary antibody to the complex. Thereafter, Enhancement Solution (available from PerkinElmer, Inc.) is added and mixed, and kept still, for example, for about 45 minutes. Then, by measuring fluorescence (excitation 340 nm/fluorescence 612 nm) by a fluorescent detector, detection or quantification is conducted.

When the methylated DNA antibody binding to methylated DNA on the detection oligonucleotide is labeled with FITC, an antibody to which FITC is bound may also be used as a secondary antibody. In this case, fluorescence of FITC may be measured by a known method to achieve detection or quantification, or detection or quantification may be achieved by using an anti-FITC antibody as a secondary antibody. Further, when FITC is directly bound to the detection oligonucleotide, FITC may be used as an identification function, or labeling function may be imparted by a horseradish Peroxidase (HRP)-labeled FITC antibody, an alkaline phosphatase-labeled FITC antibody, a biotin-labeled FITC antibody, an europium-labeled FITC antibody and the like. Concretely, as the detection oligonucleotide, when a FITC-labeled oligonucleotide is used as the detection oligonucleotide, a detection complex immobilized to a support described below containing the detection oligonucleotide is added with an antibody labeled with horseradish Peroxidase (HRP) (for example, HRP-labeled FITC antibody (available from Jackson ImmunoResearch Laboratories)), and left still at room temperature for about 1 to 2 hours, to promote binding of the FITC antibody to the detection complex. Then after washing and removing the FITC antibody solution, an appropriate substrate (for example, Substrate Reagent Pack #DY999: available from R&D SYSTEMS) is added and mixed. After leaving still at room temperature for about 5 to 60 minutes, a stop solution (2N H2SO4 aqueous solution) is added to stop the reaction of horseradish Peroxidase(HRP), and absorbance at 450 nm may be measured within 30 minutes after stopping of the reaction.

When biotin is not used for immobilization of the methylated DNA antibody, a biotinylated detection oligonucleotide can be used for detection or quantification. When a biotinylated detection oligonucleotide is detected or quantified, for example, HRP-labeled streptavidin is added and mixed with a immobilized detection complex to form and separate a conjugate of the biotinylated detection oligonucleotide and HRP-labeled streptavidin, and then activity of HRP is measured by a known method to thereby detect or quantify the biotinylated methylated DNA antibody.

The identification function may utilize a substrate used in a high sensitivity detection method such as an enzyme cycle method and the like. Concretely, an antibody to which an enzyme used in an enzyme cycle method may be bound as a detection molecule to the detection complex. The identification function imparted to the detection molecule in the present method is not limited to the methods as described above.

The "methylated DNA antibody" in the present invention is an antibody that binds to a methylated base in DNA as its antigen. Concretely, it is a methylcytosine antibody, and an antibody having a property of recognizing and binding to cytosine methylated at position 5 in single-stranded DNA can be recited. Also a commercially available methylated DNA antibody may be applicable as far as it specifically recognizes and specifically binds to DNA in a methylated state as described in the present specification. A methylated DNA antibody can be prepared by an usual immunological technique from a methylated base, methylated DNA or the like as an antigen. Concretely, for preparation of a methylcytosine antibody, it can be obtained by selecting according to specific binding to methyl cytosine in DNA as an index from an antibody that is prepared against DNA containing 5-methylcytidine, 5-methyl cytosine or 5-methyl cytosine as an antigen. Considering the property of the methylated DNA antibody (one antibody binds to one methylated base (cytosine)), it is desired to select the region where a number of methylated bases (cytosine) namely CpG exist, as the target DNA region, and improvements in quantification accuracy and detection sensitivity are expected.

As an antibody that is obtainable by immunizing an animal with an antigen, there is a method of using an antibody of IgG fraction (polyclonal antibody), and there is a method of using an antibody producing a single clone (monoclonal antibody) after immunizing with an antigen purified from an animal. In the present invention, since an antibody capable of specifically recognizing methylated DNA or methylcytosine is desired, use of a monoclonal antibody is desired.

As a method of preparing a monoclonal antibody, a procedure based on a cell fusion method can be recited. For example, in the cell fusion method, a hybridoma is prepared by allowing cell fusion between a spleen cell (B cell) derived from an immunized mouse and a myeloma cell, and an antibody produced by the hybridoma is selected for preparation of a methyl cytosine antibody (monoclonal antibody). When a monoclonal antibody is prepared by a cell fusion method, it is not necessary to purify an antigen, and for example, a mixture of 5-methyl cytidine, 5-methyl cytosine or DNA or the like containing 5-methyl cytosine may be administered as an antigen to an animal used for immunization. As an administration method, 5-methyl cytidine, 5-methyl cytosine or DNA or the like containing 5-methyl cytosine is directly administered to a mouse for production of an antibody. When an antibody is difficult to be produced, an antigen bound to a support may be used for immunization. Also, by thoroughly mixing an adjuvant solution (prepared, for example, by mixing liquid paraffin and Aracel A, and mixing killed tubercle bacilli as an adjuvant) and an antigen, and immunizing via liposome incorporating the same, immunity of an antigen can be improved. Also a method involving adding equivalent amounts of a solution containing an antigen and an adjuvant solution, fully emulsifying them, and subcutaneously or intraperitoneally injecting the resultant mixture to a mouse, and a method of adding killed Bordetella pertussis as an adjuvant after mixing well with alum water are known. A mouse may be boosted intraperitoneally or intravenously after an appropriate term from initial immunization. When the amount of an antigen is small, a solution in which the antigen is suspended may be directly injected into a mouse spleen to effect immunization. After exenterating a spleen and peeling an adipose tissue off after several days from the final immunization, a spleen cell suspension is prepared. The spleen cell is fused, for example, with an HGPRT-deficient myeloma cell to prepare a hybridoma. As a cell fusion agent, any means capable of efficiently fusing a spleen cell (B cell) and a myeloma cell is applicable, and for example, a method of using a hemagglutinating virus of Japan (HVJ), polyethyleneglycol (PEG) and the like are recited. Cell fusion may be conducted by a method using a high voltage pulse. After the cell fusion operation, cells are cultured in an HAT medium, a clone of a hybridoma in which a spleen cell and a myeloma cell are fused is selected, and the cell is allowed to grow until screening becomes possible. In a method of detecting an antibody for selecting a hybridoma that produces an intended antibody, or a method of measuring a titer of an antibody, an antigen-antibody reaction system may be used. Concretely, as a method of measuring an antibody against a soluble antigen, a radioisotope immune assay (RIA), an enzyme-linked immunosorbent assay (ELISA) and the like can be recited.

The term "specific oligonucleotide" in the present method is an oligonucleotide having a nucleotide sequence capable of binding with DNA containing a target DNA region by complementation, and has to have a function of binding with a support as will be described later. Concretely, it has a specific adhesion sequence that binds complementarily with DNA comprising a target DNA region and is able to bind with a support to form a detection complex as will be described later.

The term "specific adhesion sequence" is an oligonucleotide comprising a nucleotide sequence capable of complementarily binding with a nucleotide sequence (test oligonucleotide) comprising a target DNA region. A complementary nucleotide sequence of a nucleotide sequence part of a test oligonucleotide with which the specific adhesion sequence is able to pair has a homology of 75% or more, and preferably 90% or more with the nucleotide sequence of the specific adhesion sequence. Length of the nucleotide sequence of the specific adhesion sequence is 5 bp to 50 bp, and preferably 10 bp to 30 bp. Also the specific adhesion sequence has only to be designed so that it will have a nucleotide sequence that binds with a target DNA region or with the vicinity of the target DNA region in a test oligonucleotide, and be able to form a detection complex as will be described later in Second step. Also the specific adhesion sequence may be any nucleotide sequence as far as a detection complex comprising a detection oligonucleotide, a test oligonucleotide, a specific oligonucleotide and a support that are bound to each other can be formed, and the one that will not inhibit binding between the specific oligonucleotide and the test oligonucleotide is preferred. The specific adhesion sequence may usually be designed singly in an identical repetitive sequence (in target DNA region), but two or more may be designed. When two or more adhesion sequences are designed, preferably they will not mutually inhibit binding with DNA comprising a target DNA region.

The wording "complementarily bind" means that two single-stranded DNA form double-stranded DNA by base-pairing by a hydrogen bond between bases. For example, a base constituting single-stranded DNA and a base constituting other single-stranded DNA generate base-pairing between purine and pyrimidine, resulting that double-stranded DNA is formed by these single-stranded DNA and more concretely, double-stranded DNA is formed by base-pairing by plural sequential hydrogen bonds between thymine and adenine, and guanine and cytosine. The wording "complementarily bind" is also expressed by "complementary binding by base-pairing", "complementary base-pairing" or "bind by complementation". Nucleotide sequences that are capable of complementarily binding are also expressed by "having complementation" or "complementary" to each other. Binding of inosine contained in an artificially prepared oligonucleotide with cytosine, adenine or thymine by hydrogen bonding is also included in complementary binding. The wording "single-stranded DNA containing a nucleotide sequence that is complementary to a target DNA region" means a nucleotide sequence required for forming a bound body (double-stranded DNA) with single-stranded DNA containing a target DNA region, namely a nucleotide sequence containing a nucleotide sequence that is complementary to a part of the nucleotide sequence of the target DNA region, and is also expressed by "complementary nucleotide sequence".

In the present method, when the DNA comprising a target DNA region and the specific oligonucleotide "bind by complementation", it also includes the case where a part of the nucleotide sequence constituting the specific adhesion sequence of the specific oligonucleotide fails to base-pair with the DNA comprising a target DNA region. For example, it also includes the case that among bases constituting the specific adhesion sequence, at least 75% or more, preferably 80% or more bases base-pair with the DNA comprising a target DNA region, and able to bind with an oligonucleotide having a homology of at least 75% or more, preferably 80% or more with the DNA comprising a target DNA region.

Similarly, when DNA comprising a target DNA region and the detection oligonucleotide "bind by complementation", the case where a part of the nucleotide sequence constituting the detecting adhesion sequence of the detection oligonucleotide fails to base-pair with DNA comprising a target DNA region is also included. For example, the case where 75% or more, preferably 80% or more of the bases among the bases constituting the detecting adhesion sequence base-pair with DNA comprising a target DNA region, and are able to bind with an oligonucleotide having a homology of 75% or more, preferably 80% or more with DNA comprising a target DNA region is also included.

When the DNA comprising a target DNA region is a repetitive sequence in genome as described above, since the repetitive sequence is a group of nucleotide sequences having homology, there is a possibility that a part of the nucleotide sequence fails to base-pair with DNA comprising a target DNA region in complementary base-pairing between DNA comprising a target DNA region and a specific adhesion sequence. In the present method, when the DNA comprising a target DNA region is a repetitive sequence such as LINE sequence or SINE (Alu) sequence, a specific adhesion sequence capable of binding with a nucleotide sequence having a homology of 80% or more by complementation is included as a specific adhesion sequence.

In the present method, "nucleotide sequence showing homology" means a nucleotide sequence having sequence identity. In the present method, when the percentage of sequence homology is not described, a nucleotide sequence having a sequence identity of 75% or more, preferably 80% or more is meant. Concretely, "nucleotide sequence showing homology with SEQ ID NO:1" means the nucleotide sequence of SEQ ID NO:1 or a nucleotide sequence having a sequence identity of 75% or more with the nucleotide sequence of SEQ ID NO:1, and preferably a nucleotide sequence having a sequence identity of 80% or more.

As the "support" in the present method, the material and the shape thereof are not particularly limited as far as the later-described detection complex is bindable. For example, any shape suited for use purpose may be employed, including the shapes of tube, test plate, filter, disc, bead and the like. As the material, those used as supports for a usual immune measuring method, for example, synthetic resins such as polystyrene, polypropylene, polyacrylamide, polymethylmethacrylate, polysulfone, polyacrylonitrile and nylon, or those incorporating a sulfonic group, an amino group or the like reactive functional group in to said the synthetic resins can be recited. Also, glass, polysaccharides or derivatives thereof (cellulose, nitrocellulose and the like), silica gel, porous ceramics, metal oxides and the like may be used.

The "detection complex" means a complex wherein said test oligonucleotide, said detection oligonucleotide, said specific oligonucleotide and said support are bound. For preparing a detection complex, concretely, for example, a genomic DNA aqueous solution (0.1 pmol/10 µL, in the case of genomic DNA, it is preferred to fragment DNA by treating in advance with a suitable restriction enzyme) containing a test oligonucleotide or a test oligonucleotide aqueous solution (0.1 pmol/10 µL) is added with 5 µL of 0.02 µM biotinylated specific oligonucleotide as a specific oligonucleotide that binds with the test oligonucleotide by complementation, and added with each 5 µL of a detection oligonucleotide aqueous solution (0.02 µM) that binds with the test oligonucleotide by complementation, and further added with 20 µL of 100 mM MgCl₂, and 10 µL of an optimum 10xbuffer (330 mM Tris-Acetate pH 7.9, 660 mM KOAc, 100 mM MgOAc₂, 5 mM Dithothreitol), and then the resultant mixture is added with sterilized ultrapure water to make the liquid amount 100 µL, and then heated at 95°C for 10 minutes in the presence of a support, and retained at 70°C for 10 minutes, and further retained at 50°C for 10 minutes, and then cooled at 37°C for 10 minutes, whereby a bound body of the test oligonucleotide, the specific oligonucleotide, and the detection oligonucleotide may be obtained.

The bound body of the test oligonucleotide, the specific oligonucleotide and the detection oligonucleotide formed in this manner may be bound to a support by transferring it to an avidin plate which is a support, and keeping still for 30 minutes at room temperature. Thereafter, the remaining solution is removed and washing is executed. A washing buffer [for example, 0.05% Tween20-containing phosphate buffer (1 mM KH₂PO₄, 3 mM Na₂HPO 7H₂O, 154 mM NaCl pH7.4)] is added in a rate of 200 µL/well, and the solution is removed. This washing operation is repeated several times, to form a detection complex on the avidin plate as the support.

As a method of "binding to the support", for example, a specific oligonucleotide may be immobilized to a support according to a usual genetic engineering operation method or a commercially available kit, apparatus and the like (binding to solid phase). Concretely, there is a method of biotinylating 5'-end of a specific oligonucleotide, and then immobilizing the obtained biotinylated specific oligonucleotide to a support coated with streptavidin (for example, PCR tube coated with streptavidin, magnetic bead coated with streptavidin, chromatostrip partially coated with streptavidin and the like).

Also there is a method of covalently binding a molecule having an active functional group such as an amino group, an aldehyde group or a thiol group to 5'-end side of a specific oligonucleotide, and then letting it covalently bind to a support made of glass, silica or thermostable plastic having surface activated by a silane coupling agent or the like, for example, by a spacer formed by serially connecting five triglycerides, a cross linker or the like. Also there is a method of chemically synthesizing from the terminal side of the specific oligonucleotide directly on a support of glass or silicon.

In the above method, the test oligonucleotide, the biotinylated specific oligonucleotide and the detection oligonucleotide are added concurrently, and a bound body of the test oligonucleotide, the specific oligonucleotide and the detection oligonucleotide is obtained, and then immobilization (selection) is conducted using the biotinylated specific oligonucleotide in the bound body, however, the order is not particularly limited. In other words, a biotinylated specific oligonucleotide may be preliminarily immobilized to an avidin plate (support), and then the test oligonucleotide and the detection oligonucleotide may be added and a bound body of the test oligonucleotide, the specific oligonucleotide and the detection oligonucleotide may be obtained and immobilized (selected).

Also in obtaining and selecting a bound body of the test oligonucleotide and the detection oligonucleotide, the bound body of the test oligonucleotide and the detection oligonucleotide may be directly bound to the support by biotinylating 5'-end or 3'-end of the previously obtained test oligonucleotide and executing the method similar to the above.

In the present method, as an identification function of the detection oligonucleotide or the later-described composite detection oligonucleotide, a microparticle that is identical to the microparticle used as the support may be bound to such an oligonucleotide. As the microparticle, latex bead, gold colloid (gold nanoparticle) and the like are recited. In the present method, when microparticles of the same kind are used as the support and as the identification function of the detection oligonucleotide, the microparticle which is a support and the microparticle bound to the detection oligonucleotide can be detected as an aggregate of microparticles by immobilization of the test oligonucleotide on the microparticle which is a support, and by formation of a detection complex by the test oligonucleotide and the detection oligonucleotide by complementary binding. In this case, when the microparticle is a latex bead, the aggregate can be detected by change in turbidity. When the microparticle is a gold colloid (gold nanoparticle), the aggregate can be detected by change in color tone (pink to purple).

Further, when a plurality of nucleotide sequences that are complementary to "detecting adhesion sequence" exist on the target DNA region, supports can aggregate by binding of a plurality of detecting oligonucleotides in which a microparticle is bound to the target DNA region. In this case, an equivalent result to that obtained by adding the specific oligonucleotide can be achieved even when the specific oligonucleotide is not added. Therefore, the present method also includes a method capable of detecting aggregation of microparticles without adding a specific oligonucleotide when a plurality of nucleotide sequences capable of complementarily bind with "detecting adhesion sequence" to which the detection oligonucleotide can be bind exist on the target DNA region.

Similarly, when a plurality of nucleotide sequences that are complementary to "specific adhesion sequence" exist on the target DNA region, supports can aggregate by binding of a plurality of specific oligonucleotides to which a microparticle is bound as a support to the target DNA region. In this case, an equivalent result to that obtained by adding the detection oligonucleotide can be achieved even when the detection oligonucleotide is not added. Therefore, the present method also includes a method capable of detecting aggregation of microparticles without adding a detection oligonucleotide when a plurality of nucleotide sequences capable of complementarily binding with the specific adhesion sequence of the specific oligonucleotide on the target DNA region.

The amount detected as a microparticle aggregate can be regarded as an index correlated with an amount in the specimen of the target DNA region contained in the test oligonucleotide.

Since the "detecting adhesion sequence" and the "specific adhesion sequence" differ from each other only in that it is a nucleotide sequence contained in the detection oligonucleotide or it is a nucleotide sequence contained in the specific oligonucleotide, the detection oligonucleotide and the specific oligonucleotide are respectively oligonucleotides capable of concurrently binding with a test oligonucleotide. In other words, the "detecting adhesion sequence" may also be used as the "specific adhesion sequence", and the "specific adhesion sequence" may also be used as the "detecting adhesion sequence".

For example, concretely, by setting the nucleotide sequence of SEQ ID NO:4 and the nucleotide sequence of SEQ ID NO:12, respectively as the "specific adhesion sequence" and the "detecting adhesion sequence" capable of binding with SEQ ID NO:1, biotinylating the specific adhesion sequence of SEQ ID NO:4 to make it a specific oligonucleotide, and combining a detection sequence in continuous with the detecting adhesion sequence of SEQ ID NO:12 to prepare the detection oligonucleotide of SEQ ID NO:5, it is possible to detect the nucleotide sequence of SEQ ID NO:1 by using the specific oligonucleotide and the detection oligonucleotide. On the other hand, by setting the nucleotide sequence of SEQ ID NO:12 and the nucleotide sequence of SEQ ID NO:4, respectively as the "specific adhesion sequence" and the "detecting adhesion sequence" capable of binding with the SEQ ID NO:1, biotinylating the specific adhesion sequence of SEQ ID NO:12 to make it a specific oligonucleotide, and combining a detection sequence in continuous with the nucleotide sequence of the detecting adhesion sequence of SEQ ID NO:5 to prepare a detection oligonucleotide, it is possible to similarly detect the nucleotide sequence of SEQ ID NO:1 by using the specific oligonucleotide and the detection oligonucleotide. Similarly, as a combination of the "specific adhesion sequence" and the "detecting adhesion sequence" capable of binding with SEQ ID NO:1, the nucleotide sequence of SEQ ID NO:8 and the nucleotide sequence of SEQ ID NO:14 can be recited; as a combination of the "specific adhesion sequence" and the "detecting adhesion sequence" capable of binding with SEQ ID NO:2, the nucleotide sequence of SEQ ID NO:6 and the nucleotide sequence of SEQ ID NO:13 can be recited; and as a combination of the "specific adhesion sequence" and the "detecting adhesion sequence" capable of binding with SEQ ID NO:3, the nucleotide sequence of SEQ ID NO:10 and the nucleotide sequence of SEQ ID NO:15 can be recited.

The "specific adhesion sequence" and the "detecting adhesion sequence" that constitute the specific oligonucleotide and the detection oligonucleotide capable of forming a detection complex by binding to the test oligonucleotide, can be treated as a combination of the nucleotide sequences capable of complementarily binding with the test oligonucleotide capable of forming a detection complex, and when either one of the combination is used as the specific adhesion sequence, the other of the combination may be used as the detecting adhesion sequence.

The "composite detection oligonucleotide" in the present method is a nucleotide complementarily binding with a test oligonucleotide, to which a plurality of oligonucleotides having an identification function are bound (composited). As a form of this composite detection oligonucleotide, it may be formed by binding of adhesion nucleotide sequences comprising mutually complementary nucleotide sequences possessed by the respective oligonucleotides, and all or part of each oligonucleotide constituting the composite detection oligonucleotide may be methylated. In the present method, the one in which at least a part of the composite detection oligonucleotide is methylated is also called a methylated composite detection oligonucleotide. Also, the methylated composite detection oligonucleotide may be a composite detection oligonucleotide containing a methylated oligonucleotide. When a composite detection oligonucleotide is formed by complementary binding a methylated oligonucleotide, the resultant methylated composite detection oligonucleotide may be, for example, the one to which only a methylated oligonucleotide is bound or the one to which combination of a methylated oligonucleotide and an unmethylated (non-methylated) oligonucleotide is bound, and the numbers of respective oligonucleotides are not particularly limited.

The "methylated oligonucleotide" is an oligonucleotide in which a base of a nucleotide constituting the oligonucleotide is methylated, and may be an artificially synthesized one in the present invention. Also, it may be prepared by modifying an artificially synthesized oligonucleotide or an oligonucleotide obtained by fragmentation of genomic DNA with a methyl group transferase. Some methyl group transferases (methyltransferase) are known to methylate position 5 of cytosine in "CpG" in an oligonucleotide, and concrete examples of such a methyl group transferase include SssI methylase, Dmnt1 methylase and on the like. Here, since genomic DNA is partially methylated, it is sometimes the case that a region methylated in genome can be obtained as a methylated oligonucleotide by fragmentation of genomic DNA obtained from a cell or the like. Also, the methylated oligonucleotide in which position 5 of cytosine is methylated may be a methylated oligonucleotide that is artificially synthesized by using 5-methylcytosine in place of cytosine. In this case, not only cytosine in "CpG", but also all cytosines (for example, 5'-CA-3', 5'-CT- 3', 5'-CC- 3' and the like) may be synthesized as 5-methylcytosine.

The "DNA methylation enzyme" means an enzyme that methylates a base in DNA, and various kinds DNA methylation enzymes are isolated from mammalian cells, bacteria and the like. DNA methylation enzymes are classified into several kinds such as adenine methylation enzymes, and cytosine methylation enzymes according to the kind of the base of a substrate. A cytosine methylation enzyme is an enzyme that recognizes a specific sequence in a DNA nucleotide sequence, and methylates cytosine near the sequence, and different cytosine methylation enzymes are known according to the recognized nucleotide sequences.

A number of methylation reactions of DNA catalyzed by a DNA methylation enzyme are found from a primitive immune system called a restriction-modification system. The restriction-modification system is a function that digests foreign DNA (in particular, bacteriophage) with a restriction enzyme after regularly methylating the entire genome functioning in bacteria to protect it from being digested by a restriction enzyme (restriction endonuclease) that recognizes a specific sequence, and is a system for protecting a microbial genome from bacteriophage infection. Enzymes functioning in methylation of genome are known to methylate cytosine or adenine, and often known to methylate nitrogen at position 6 (N6) or carbon at position 5 (C5) of a purine residue. Among these enzymes, known as a cytosine methylation enzyme that methylates C5 of cytosine are SssI (M.SssI) methylase, AluI methylase, HhaI methylase, HpaII methylase, MspI methylase, HaeIII methylase, and so on. These enzymes that methylate position C5 of cytosine recognize different nucleotide sequences, and a cytosine methylation enzyme that recognizes CpG is only SssI.

As a methylation reaction of DNA in human genome, methylation at position 5 (C5) of cytosine in CpG is known as epigenetics (the mechanism generating diversity of gene expression independent of gene sequence), and as such a cytosine methylation enzyme, DNA methyltransferase is known. As a DNA methyltransferase, DnmtI methyltransferase is known.

In human cells, since position C5 of cytosine in a CpG sequence is methylated, for methylating genome artificially, the same position of the same cytosine in the same sequence (CpG) with methylation in a human cell can be methylated by using SssI.

For making methylated DNA by a cytosine methyltransferase, concretely, for example, the following operation may be conducted. A DNA sample is added with 5 µL of an optimum 10×buffer (NEBuffer2 (available from NEB)), 0.5 µL of S-adenosyl methionine (3.2 mM, available from NEB), and 0.5 µL of cytosine methyltransferase SssI (available from NEB) respectively, and the resultant mixture is added with sterilized ultrapure water to make the liquid amount 50 µL, and then incubated at 37°C for 30 minutes.

In the present method, when the oligonucleotides constituting the composite detection oligonucleotide mutually complementarily bind (link) in series (also called "serial type"), the oligonucleotide having a detecting adhesion sequence binding with the test oligonucleotide by complementation is called a first oligonucleotide among the oligonucleotides constituting the composite detection oligonucleotide (including a methylated oligonucleotide).

The first oligonucleotide has a first adhesion nucleotide sequence which is an adhesion nucleotide sequence having said detecting adhesion sequence, and capable of complementarily binding with a complementary adhesion sequence of a second oligonucleotide which is an oligonucleotide (including methylated oligonucleotide) capable of complementarily binding with the first oligonucleotide while not complementarily binding with nucleotide sequences other than said detecting adhesion sequence of the test oligonucleotide.

Further, an oligonucleotide (including methylated oligonucleotide) having a complementary first adhesion nucleotide sequence comprising a nucleotide sequence capable of complementarily binding with the first adhesion nucleotide sequence is called a second oligonucleotide. The second oligonucleotide has a second adhesion nucleotide sequence which is an adhesion nucleotide sequence having said complementary first detecting adhesion sequence, and capable of complementarily binding with a third oligonucleotide which is an oligonucleotide (including methylated oligonucleotide) capable of complementarily binding with the second oligonucleotide while not complementarily binding with the test oligonucleotide other than the first adhesion sequence sequence and the first oligonucleotide. An oligonucleotide (including methylated oligonucleotide) having a complementary second adhesion nucleotide sequence comprising a nucleotide sequence capable of complementarily binding with the second adhesion nucleotide sequence is called a third oligonucleotide.

Similarly, an oligonucleotide (including methylated oligonucleotide) having a complementary Nth adhesion nucleotide sequence comprising a nucleotide sequence capable of complementarily binding with an Nth adhesion nucleotide sequence is called a (N+1)th oligonucleotide. The (N+1)th oligonucleotide has a (N+1)th adhesion nucleotide sequence which is an adhesion nucleotide sequence having the complementary (N)th adhesion nucleotide sequence, and capable of complementarily binding with a (N+2)th oligonucleotide which is an oligonucleotide (including methylated oligonucleotide) capable of complementarily binding with the (N+1)th oligonucleotide while not complementarily binding with the test oligonucleotide other than the Nth adhesion sequence and oligonucleotides from the first oligonucleotide to the Nth oligonucleotide. An oligonucleotide (including methylated oligonucleotide) having a complementary (N+1)th adhesion nucleotide sequence comprising a nucleotide sequence capable of complementarily binding with the (N+1)th adhesion nucleotide sequence is called a (N+2)th oligonucleotide.

Similarly, an oligonucleotide (including methylated oligonucleotide) having a complementary (N-1)th adhesion nucleotide sequence comprising a nucleotide sequence capable of complementarily binding with the (N-1)th adhesion nucleotide sequence is called a Nth oligonucleotide. The Nth oligonucleotide has a Nth adhesion nucleotide sequence which is an adhesion nucleotide sequence having the complementary (N-1)th adhesion nucleotide sequence, and capable of complementarily binding with the (N+1)th oligonucleotide which is an oligonucleotide (including methylated oligonucleotide) capable of complementarily binding with the Nth oligonucleotide while not complementarily binding with the test oligonucleotide other than the (N-1)th adhesion sequence and oligonucleotides from the first oligonucleotide to the (N-1)th oligonucleotide.

When the (N+1)th oligonucleotide does not exist, the Nth oligonucleotide is called a terminal oligonucleotide, and the Nth oligonucleotide may not have an Nth adhesion nucleotide sequence.

That is, one form of the composite detection oligonucleotide in the present invention is such that oligonucleotides from the first oligonucleotide to the terminal oligonucleotide are linked by complementary binding between an adhesion nucleotide sequence and a complementary adhesion nucleotide sequence. When the composite detection oligonucleotide is formed only of the first oligonucleotide, the first oligonucleotide may not have an adhesion nucleotide sequence as described above, and further may be a methylated oligonucleotide.

The adhesion nucleotide sequence and the complementary adhesion nucleotide sequence may be nucleotide sequences to which an oligonucleotide (including methylated oligonucleotide) is capable of binding complementarily, and may be situated at a terminal end or in the middle of the oligonucleotide.

Further, the Nth adhesion nucleotide sequence fails to complementarily bind with nucleotide sequences other than the complementary Nth adhesion nucleotide sequence, and it is desired that the Nth nucleotide sequence does not inhibit any complementary binding other than the complementary Nth adhesion nucleotide sequence. It is desired that the Nth adhesion nucleotide sequence fails to complementarily bind with nucleotide sequences of oligonucleotides including an oligonucleotide constituting a composite detection oligonucleotide, nucleic acid contained in a specimen, a test oligonucleotide and a specific oligonucleotide other than the complementary Nth adhesion nucleotide sequence.

In the present method, when oligonucleotides constituting a composite detection oligonucleotide can be branched (other than serially) complementarily to each other and bound (linked) (also called "branched"), a plurality of adhesion nucleotide sequences may exist on the Nth oligonucleotide among the oligonucleotides constituting the composite detection oligonucleotide (including methylated oligonucleotide). For example, when there are M adhesion nucleotide sequences in the Nth oligonucleotide, they are called a (N,1)th adhesion nucleotide sequence, a (N,2)th adhesion nucleotide sequence, a (N,3)th adhesion nucleotide sequence, ···, a (N,(M-1))th adhesion nucleotide sequence, and a (N,M) adhesion nucleotide sequence respectively, and oligonucleotides that bind with these nucleotide sequences by complementation are respectively called a ((N+1),1)th oligonucleotide, a ((N+1),2)th oligonucleotide, a ((N+1),3)th oligonucleotide, ···, a ((N+1), (N-1))th oligonucleotide, and a ((N+1),M)th oligonucleotide. In this case, for example, when there is no ((N+2),1)th oligonucleotide, the ((N+1),1)th oligonucleotide is a terminal oligonucleotide, and the ((N+1),1)th adhesion nucleotide sequence may not exist.

Further, similarly, when there are a plurality of adhesion nucleotide sequences on a (N,1)th oligonucleotide, for example, when there are L adhesion nucleotide sequences on the (N,1)th oligonucleotide, they are called a (N,1,1)th adhesion nucleotide sequence, a (N,1,2)th adhesion nucleotide sequence, a (N,1,3)th adhesion nucleotide sequence, ···, a (N,1,(L-1))th adhesion nucleotide sequence, and a (N,1,L)th adhesion nucleotide sequence, respectively, and oligonucleotides that bind with these adhesion nucleotide sequences by complementation are respectively called a ((N+1),1,1)th oligonucleotide, a ((N+1),1,2)th oligonucleotide, a ((N+1),1,3)th oligonucleotide, ···, a ((N+1),1,(L-1))th oligonucleotide, and a ((N+1),1,L)th oligonucleotide. In this case, for example, when there is no ((N+2),1,1)th oligonucleotide, the ((N+1),1,1)th oligonucleotide is a terminal oligonucleotide, and the ((N+1),1,1)th adhesion nucleotide sequence may not exist.

The branched composite oligonucleotide includes not only the case where the composite detection oligonucleotide is a branched type, but also the case where plural kinds of first oligonucleotides exist, and oligonucleotides (including methylated oligonucleotide) which are the plural kinds of first oligonucleotides bind on the test oligonucleotide. In this case, when there are M first oligonucleotides on the test oligonucleotide, a (1,1)th oligonucleotide, a (1,2)th oligonucleotide, a (1,3)th oligonucleotide, ···, and a (1,M)th oligonucleotide having a (1,1)th adhesion sequence, a (1,2)th adhesion sequence, a (1,3)th adhesion sequence, ···, and a (1,M)th adhesion sequence capable of complementarily binding with a first linkage sequence, a second linkage sequence, ···, and a Mth linkage sequence on the test oligonucleotide can be recited. When there are M second oligonucleotides on the first oligonucleotide, they may be a (2,1)th oligonucleotide, a (2,2)th oligonucleotide, a (2,3)th oligonucleotide, ···, and a (2,M)th oligonucleotide respectively having a (2,1)th adhesion sequence, a (2,2)th adhesion sequence, a (2,3)th adhesion sequence, ···, and a (2,M)th adhesion sequence respectively capable of complementarily binding with a first linkage sequence, a second linkage sequence, ···, and a Mth linkage sequence on the first oligonucleotide can be recited.

Further, when there are M N+1th oligonucleotides on the Nth oligonucleotide, they may be a (N+1,1)th oligonucleotide, a (N+1,2)th oligonucleotide, a (N+1,3)th oligonucleotide, ···, and a (N+1,M)th oligonucleotide respectively having a (N+1,1)th adhesion sequence, a (N+1,2)th adhesion sequence, a (N+1,3)th adhesion sequence, ···, and a (N+1,M)th adhesion sequence, respectively capable of binding with the first linkage sequence, the second linkage sequence, ···, and the Mth linkage sequence on the Nth oligonucleotide.

When there are P N+1th oligonucleotides on the (N,M)th oligonucleotide, they may be a (N+1,M,1)th oligonucleotide, a (N+1,M,2)th oligonucleotide, a (N+1,M,3)th oligonucleotide, ···, and a (N+1,M,P)th oligonucleotide, respectively having a (N+1,M,1)th adhesion sequence, a (N+1,M,2)th adhesion sequence, a (N+1,M,3)th adhesion sequence, ···, and a (N+1,M,P)th adhesion sequence, respectively capable of complementarily binding with the (N+1,M,1)th linkage sequence, the (N+1,M,2)th linkage sequence, ···, and the (N+1,M,P)th linkage sequence on the (N,M)th oligonucleotide.

Further, when there are P N+1th oligonucleotides on the (N,M,···,X)th oligonucleotide, they may be a (N+1,M,···,X,1)th oligonucleotide, a (N+1,M,···,X,2)th oligonucleotide, and a (N+1,M,···,X,3)th oligonucleotide, ···, and a (N+1,M,···,X,P)th oligonucleotide respectively having a (N+1,M,···,X,1)th adhesion sequence, a (N+1,M,···,X,2)th adhesion sequence, and a (N+1,M,···,X,3)th adhesion sequence, ···, and a (N+1,M,···,X,P)th adhesion sequence, respectively capable of complementarily binding with the (N+1,M,···, X,1)th linkage sequence, the (N+1,M,···,X,2)th linkage sequence, ···, and the (N+1,M,···,X,P) th linkage sequence on the (N,M,···,X)th oligonucleotide.

As described above, it is possible to improve the sensitivity of the composite detection oligonucleotide by using various combinations, and each combination of an oligonucleotide and a terminal oligonucleotide can be adjusted depending on the sensitivity or accuracy with which detection is intended to be made.

When there are a plurality of adhesion nucleotide sequences on one oligonucleotide, these adhesion nucleotide sequences may be identical or different. Concretely, for example, nucleotide sequences of said (N,1)th adhesion nucleotide sequence, (N,2)th adhesion nucleotide sequence, and (N,3)th adhesion nucleotide sequence may be identical, or may be nucleotide sequences that are different from each other. It suffices that the linkage adhesion sequence and the complementary linkage nucleotide sequence, and the adhesion nucleotide sequence and the linkage nucleotide sequence may be nucleotide sequences that are able to complementarily bind with each other, and it suffices that they have a homology of 90% or more, and usually 5 to 100 bp, and preferably 10 to 50 bp. Preferably, the nucleotide sequence is designed so that it will fail to complementarily bind with genome, and more preferably, it is artificially synthesized DNA. For confirming that the designed adhesion nucleotide sequence, linkage nucleotide sequence or the like fails to complementarily bind with genome in a simple and convenient manner, Blast searching may be executed using a genome database of a public institution such as PubMeD to determine that there is no nucleotide sequence showing a homology of 80% or more.

That is, the "composite detection oligonucleotide" is linked to the test oligonucleotide by complementary binding between the linkage nucleotide sequence and the complementary linkage nucleotide sequence, to form a detection complex. The detection complex may be the one in which one detection oligonucleotide is bound to one test oligonucleotide, or may be the one in which a plurality of composite detection oligonucleotides are bound. The composite detection oligonucleotide may be a methylated composite detection oligonucleotide. When a plurality of composite detection oligonucleotides bind to one test oligonucleotide, individual composite detection oligonucleotides may be identical composite detection oligonucleotides, or may be different composite detection oligonucleotides. The linkage nucleotide sequence on the test oligonucleotide may be each one of several kinds of linkage nucleotide sequences, or a plurality of one kind of linkage nucleotide sequences.

The "test oligonucleotide which is DNA comprising a target DNA region" obtained in First step of the present invention may be single-stranded DNA. The method for obtaining the test oligonucleotide as single-stranded DNA from a specimen may be any method. Concretely, when the target DNA region is a genomic DNA aqueous solution (0.1 pmol/10µL, In the case of genomic DNA, it is desired to treat in advance with an appropriate restriction enzyme to fragment the DNA.) or a test oligonucleotide aqueous solution (0.1 pmol/10 µL) is added with 10 µL of an optimum 10×buffer (330 mM Tris-Acetate pH 7.9, 660 mM KOAc, 100 mM MgOAc₂, 5 mM Dithothreitol), and then the resultant mixture is added with sterilized ultrapure water to make the liquid amount 100 µL, and heated at 95°C for 10 minutes, and rapidly cooled at 4°C. The liquid amount may be adjusted to 100 µL only with sterilized ultrapure water without using a buffer, and heated at 95°C for 10 minutes and rapidly cooled at 4°C. Further, when the target DNA region is DNA that is possibly single-stranded DNA or a hybrid with RNA in a specimen, such as viral genomic DNA (single-stranded DNA) or DNA synthesized from RNA by a reverse transcriptase, it is desired to heat at 95°C for 10 minutes in a solution of a buffer or sterilized ultrapure water and rapidly cool at 4°C likewise the above.

In the present method, to "form a detection complex comprising the test oligonucleotide, the detection oligonucleotide, the specific oligonucleotide and the support", for example, a genomic DNA aqueous solution containing a test oligonucleotide (0.1 pmol/10 µL, In the case of genomic DNA, it is desired to previously treat with an appropriate restriction enzyme to fragment the DNA.) or a test oligonucleotide aqueous solution (0.1 pmol/10 µL) is added with 5 µL of 0.02 µM biotinylated specific oligonucleotide as a specific oligonucleotide binding with the test oligonucleotide by complementation, 5 µL of a detection oligonucleotide aqueous solution (0.02 µM) respectively, binding with the test oligonucleotide by complementation, 20 µL of 100 mM MgCl₂, and 10 µL of an optimum 10×buffer (330 mM Tris-Acetate pH 7.9, 660 mM KOAc, 100 mM MgOAc₂, 5 mM Dithothreitol), and then the resultant mixture is added with a sterilized ultrapure water to make the liquid amount 100 µL, and then heated at 95°C for 10 minutes, retained at 70°C for 10 minutes, and retained at 50°C for 10 minutes, and then cooled at 37°C for 10 minutes.

The bound body of the test oligonucleotide, the specific oligonucleotide and the detection oligonucleotide thus formed is transferred to an avidin plate which is a support, and kept still for 30 minutes at room temperature, whereby a detection complex can be obtained. Thereafter, the remaining solution is removed and washing is conducted. A washing buffer [for example, 0.05% Tween20-containing phosphate buffer (1 mM KH₂PO₄, 3 mM Na₂HPO 7H₂O, 154 mM NaCl pH7.4)] is added in a rate of 200 µL/well, and the solution is removed. This washing operation is repeated several times, to obtain a detection complex on an avidin plate as a support.

Third step is a step of quantifying or detecting said DNA comprising a target DNA region by detecting the detection oligonucleotide contained in the detection complex formed in Second step by its identification function. As a method of "quantifying or detecting said DNA comprising a target DNA region by detecting the detection oligonucleotide contained in the detection complex formed in Second step by its identification function" which is Third step, concretely, for example, when said detection oligonucleotide is a methylated oligonucleotide, a methylated DNA antibody is added as a detection molecule to the avidin plate to which the bound body of said test oligonucleotide, the specific oligonucleotide and the detection oligonucleotide is bound, and allowed to bind to the detection oligonucleotide contained in the detection complex. Thereafter, the remaining solution is removed and washing is conducted using a washing buffer to leave the detection complex. More concretely, for example, the plate on which the bound body of the test oligonucleotide, the specific oligonucleotide and the detection oligonucleotide obtained in the concrete example of the method as described above is immobilized is added with a methylcytosine antibody (1 mg/mL) prepared to be 1 µg/mL in a rate of 100 µL/well, and kept still for 1 hour at room temperature. Thereafter, the remaining solution is removed and washing is executed. A washing buffer [for example, 0.05% Tween20-containing phosphate buffer (1 mM KH₂PO₄, 3 mM Na₂HPO 7H₂O, 154 mM NaCl pH7.4)] is added in a rate of 200 µL/well, and the solution is removed. This washing operation is repeated several times, to leave a methylcytosine antibody bound to the detection complex.

Besides the above, concretely, for example, to a detection complex to which a methylated DNA antibody is bound, an antibody (secondary antibody) labeled with europium (hereinafter, somtimes described as "Eu") that binds to the methylated DNA antibody is allowed to bind, and after adding Enhancement solution (available from PerkinElmer, Inc.), fluorescence at excitation 340 nm/fluorescence 612 nm may be measured. FITC label may be used in place of Eu label. To the antibody (secondary antibody) labeled with FITC, a FITC antibody labeled with HRP may further be bound, and detection may be made by enzyme activity of HRP. When detection is made using enzyme activity of HRP, after adding a substrate (R&D systems, Inc., #DY999) and incubating at room temperature, Stop solution (1M H₂SO₄: 50 µL/well) may be added, and absorbance at 450 nm (Reference 650 nm) may be measured.
Also, an antibody to which a substrate detectable by an enzyme cycle method may be used as a secondary antibody.

More concretely, to the detection complex (the support is an avidin plate) to which the methylated DNA antibody obtained in the foregoing concrete example of method is bound, a mouse IgG antibody Eu-N1 (available from PerkinElmer, Inc.) prepared into 0.25 µg/mL is added in a rate of 100 µL/well, and kept still for 1 hour at room temperature, and then the remaining solution is removed, and a washing buffer [for example, 0.05% Tween20-containing phosphate buffer (1 mM KH₂PO₄, 3 mM Na₂HPO 7H₂O, 154 mM NaCl pH7.4)] is added in a rate of 200 µL/well, and the solution is removed. This washing operation is repeated several times. Enhancement solution is added in an amount of 200 µL/well, and incubated at room temperature for 45 minutes after stirring. Sequentially, measurement is conducted at excitation 340 nm/fluorescence 612 nm (Lag Time 400msec, Integration 400 msec).

To the detection complex (the support is an avidin plate) to which the methylated DNA antibody obtained in the foregoing concrete example of method is bound, a mouse IgG antibody (goat) labeled with FITC prepared into 2 µg/mL is added in a rate of 100 µL/well, and kept still for 1 hour at room temperature, and the remaining solution is removed and a washing buffer [for example, 0.05% Tween20-containing phosphate buffer (1 mM KH₂PO₄, 3 mM Na₂HPO 7H₂O, 154 mM NaCl pH7.4)] is added in a rate of 200 µL/well, and the solution is removed. This washing operation is repeated several times. Further, a secondary antibody against FITC (for example, HRP-labeled anti FITC antibody:available from Jackson ImmunoResearch Laboratories, Inc.) is added in a rate of 100 µL/well to the avidin plate, and incubated at room temperature. A washing buffer [for example, 0.05% Tween20-containing phosphate buffer (1 mM KH₂PO₄, 3 mM Na₂HPO 7H₂O, 154 mM NaCl pH7.4)] is added in a rate of 200 µL/well, and the solution is removed. This washing operation is repeated several times. A substrate (R&D Systems, Inc., #DY999) is added in a rate of 100 µL/well, and stirred for about 10 seconds. After incubating at room temperature, Stop solution (1M H₂SO₄: 50 µL/well) is added and stirred for about 10 seconds. In 30 minutes, absorbance at 450 nm (Reference 650 nm) is measured (light shielding is preferred).

The present invention may be used in the following situations.

For example, in diagnosis of cancer or the like, it may be used as a screening test in a regular health examination by quantification of free DNA in blood. In diagnosis of infection or the like, by quantifying or detecting DNA or DNA prepared from RNA as a template of a bacterium or virus which is a cause of the disease, a simple method for identifying a causative bacterium or a causative virus of infection, or a food poisoning bacterium can be provided. In conventional techniques, free DNA in blood, DNA derived from a microorganism, or DNA synthesized from RNA as a template is quantified or detected after amplifying DNA by conducting PCR or the like. However, by using the present method, DNA quantification or detection is enabled without conducting a complicated method such as PCR.

As a method of detecting or quantifying protein or low molecular biological substances contained in a biological sample such as blood or urine, immunological measuring methods are generally used. Among such immunological measuring methods, a so-called immune chromatography using chromatography is widely used in various situations including, for example, clinical examinations in hospitals, assays in laboratories and the like because of its simple operation and short time required for assay. In recent years, a so-called hybrid chromatography has been utilized wherein labeled DNA (gene) is developed on a chromatostrip, and target DNA (gene) is detected by hybridization using a probe capable of capturing the target DNA (gene). Also this method is now coming to be widely used in situations including, for example, clinical examinations in hospitals, assays in laboratories and the like because of its simple operation and short required time for assay. The present measurement method conceptually enables a combined method of the immune chromatography and the hybrid chromatography. In the present method, since the order of formation of a complex and selection of a complex is not particularly limited, various methods are possible. Concretely, such methods may be executed in the following manner.

For example, to a sample directly after end of Second step, a biotinylated specific oligonucleotide and a detection oligonucleotide having an identification function are added, and the methylated single-stranded DNA containing a target DNA region, the detection oligonucleotide having an identification function and the biotinylated specific oligonucleotide are allowed to bind each other, to thereby form a detection complex in which a bound body of the single-stranded DNA containing a target DNA region, the detection oligonucleotide having an identification function, and the biotinylated specific oligonucleotide is bound to the support. As the obtained sample is dropped (applied) into an applying part of a chromatostrip, said detection complex migrates in a development part by a capillary phenomenon, and is trapped in the part preliminarily coated with streptavidin. Then by detecting or quantifying the detection oligonucleotide contained in the obtained complex according to its identification function, DNA comprising a target DNA region can be detected or quantified.

It is also possible to set a nucleotide sequence capable of complementarily binding with a plurality of detecting nucleotide sequences (using a detection oligonucleotide respectively capable of complementarily binding with different nucleotide sequences on the target DNA region) as the target DNA region, and detect or quantify each target DNA region sequentially. Also, detection sensitivity can be dramatically improved by using a detection oligonucleotide capable of complementarily binding with a target DNA region existing plurally in genome, that detects a repetitive sequence in genome, a duplicate gene or a plurality of different genes concurrently so as to enable formation of a detection complex with a plurality of target DNA regions.

When there are a plurality of DNA comprising a plurality of target DNA regions, if formation of a bound body of each test oligonucleotide comprising a target DNA region, each specific oligonucleotide complementarily binding with the test oligonucleotide and a detection oligonucleotide is possible even in the presence of other test oligonucleotide, specific oligonucleotide and detection oligonucleotide, DNA comprising a plurality of target DNA regions can be detected concurrently.

The detection oligonucleotide and the specific oligonucleotide have any of the nucleotide sequences as shown below:
(1) the nucleotide sequence of SEQ ID NO:1, or a nucleotide sequence having 80% or more sequence identity to the same;
(2) a complementary sequence to the nucleotide sequence of SEQ ID NO:1, or a nucleotide sequence having 80% or more sequence identity to the same;
(3) the nucleotide sequence of SEQ ID NO:2, or a nucleotide sequence having 80% or more sequence identity to the same;
(4) a complementary sequence to the nucleotide sequence of SEQ ID NO:2, or a nucleotide sequence having 80% or more sequence identity to the same;
(5) the nucleotide sequence of SEQ ID NO:3, or a nucleotide sequence having 80% or more sequence identity to the same;
(6) a complementary sequence to the nucleotide sequence of SEQ ID NO:3, or a nucleotide sequence having 80% or more sequence identity to the same;
(7) the nucleotide sequence of SEQ ID NO:4, or a nucleotide sequence having 80% or more sequence identity to the same;
(8) a complementary sequence to the nucleotide sequence of SEQ ID NO:4, or a nucleotide sequence having 80% or more sequence identity to the same;
(9) the nucleotide sequence of SEQ ID NO:5, or a nucleotide sequence having 80% or more sequence identity to the same;
(10) a complementary sequence to the nucleotide sequence of SEQ ID NO:5, or a nucleotide sequence having 80% or more sequence identity to the same;
(11) the nucleotide sequence of SEQ ID NO:6, or a nucleotide sequence having 80% or more sequence identity to the same;
(12) a complementary sequence to the nucleotide sequence of SEQ ID NO:6, or a nucleotide sequence having 80% or more sequence identity to the same;
(13) the nucleotide sequence of SEQ ID NO:7, or a nucleotide sequence having 80% or more sequence identity to the same;
(14) a complementary sequence to the nucleotide sequence of SEQ ID NO:7, or a nucleotide sequence having 80% or more sequence identity to the same;
(15) the nucleotide sequence of SEQ ID NO:8, or a nucleotide sequence having 80% or more sequence identity to the same; and
(16) a complementary sequence to the nucleotide sequence of SEQ ID NO:8, or a nucleotide sequence having 80% or more sequence identity to the same.

For example, a nucleotide sequence having a sequence homology of 80% or more with the nucleotide sequence of SEQ ID NO:1 has about 280 copies in a human genome, a nucleotide sequence having a sequence homology of 80% or more with the nucleotide sequence of SEQ ID NO:2 has about 260 copies in a human genome and a nucleotide sequence having a sequence homology of 80% or more with the nucleotide sequence of SEQ ID NO:3 has about 820 copies in a human genome. Therefore, if one can set a detecting adhesion sequence and a specific adhesion sequence in each nucleotide sequence, the detection sensitivity of one genome can be improved to 280 to 820 folds theoretically, compared to the case where a detecting adhesion sequence and a specific adhesion sequence are set for a sequence having just one kind in genome.

As a method of executing the process of obtaining a detection complex by binding a bound body of the detection oligonucleotide, the biotinylated methylated antibody, and the test oligonucleotide which is a target DNA region or DNA prepared from a target RNA region, to the support, any method using an immunological antibody method can be used without limited to the methods as described above. For example, in the ELISA method, since it uses the principle similar to that of the chromatostrip method, the process of forming the bound body of the test oligonucleotide, the specific oligonucleotide and the detection oligonucleotide, and making it to bind with the support can be executed in the described order.

In First step of the present method, it is preferred to extract DNA from a specimen by a system containing a sodium salt at high concentration. Concretely, as a concentration of sodium salt in a solution (for example, buffer) used in a DNA extraction operation for obtaining DNA from a specimen in First step of the present method, at least 50 mM or more, and preferably 100 mM or more can be recited. More concretely, 50 mM or more and 1000 mM or less, preferably 100 mM or more and 1000 mM or less, more preferably 100 mM or more and 200 mM or less can be recited. Any salts including NaCl, NaCO₃, Na₂SO₄ and the like are applied as far as it is a salt containing a sodium ion, and preferably means NaCl.

The present invention is a method of selecting a specimen derived from a cancer patient, and includes the steps of evaluating a specimen derived from a test subject as a specimen derived from a cancer patient when there is a significant difference between a DNA quantification result or detection result quantified or detected using a specimen derived from a test subject by the method according to any one of Inventions 1 to 13, and a DNA quantification result or detection result quantified or detected using a specimen derived from a healthy subject by the method, and identifying the specimen derived from a cancer patient based on the evaluation result. As a preferred aspect of the present invention, the invention in which the specimen is a serum derived from a mammal, and the invention in which the DNA comprising a target DNA region is free DNA comprising a target DNA region in serum derived from a mammal can be recited. Use of these inventions will make it possible to identify a cancer patient in a simple and convenient manner by a blood test.

Here, the "cancer patient" is a test subject developing a cancer, and as the cancer, solid cancers developing in organs of human and mammals, and non-solid cancers developing in blood of human and mammals such as lung cancer (non-small-cell lung cancer, small-cell lung cancer), esophageal cancer, gastric cancer, duodenal cancer, colon cancer, rectal cancer, hepatic cancer (hepatocarcinoma, cholangiocellular carcinoma), gallbladder cancer, bile duct cancer, pancreatic cancer, colon cancer, anal cancer, breast cancer, cervical cancer, uterine cancer, ovarian cancer, vulvar cancer, vaginal cancer, prostate cancer, kidney cancer, ureter cancer, bladder cancer, prostate cancer, penile cancer, testicular (testis) cancer, maxillary cancer, tongue cancer, (naso-, oro-, hypo-) pharyngeal cancer, acute myeloid leukemia, chronic myelogenous leukemia, acute lymphoblastic leukemia, chronic lymphoblastic leukemia, malignant lymphoma, myelodysplastic syndrome, thyroid cancer, brain tumor, osteosarcoma and skin cancer (basal cell cancer, squamous cell cancer) are included.

The methylated oligonucleotide or the like in the present invention is useful as a reagent of a detection kit. The scope of the present method includes use in the form of a detection kit or a detection chip as described above using the substantial principle of the present invention.

### EXAMPLES

In the following, the present invention will be described in detail by way of examples, however, the present invention is not limited to these examples.

### Example 1

The following TE buffer solutions were prepared in duplicate for each genomic DNA derived from human blood purchased from Clontech, Inc.
Solution A: genomic DNA derived from human blood 500 ng/20 µL TE buffer solution
Solution B: genomic DNA derived from human blood 50 ng/20 µL TE buffer solution
Solution C: genomic DNA derived from human blood 5 ng/20 µL TE buffer solution
Solution D: genomic DNA derived from human blood 0 ng/20 µL TE buffer solution (negative control solution)

Twenty (20) µL of each solution prepared above, 10U of restriction enzyme XspI, and 5 µL of 10x buffer optimum for XspI (200 mM Tris-HCl pH 8.5, 100 mM MgCl₂, 10 mM Dithiothreitol, 1000 mM KCl) were mixed, and added with sterilized ultrapure water to make the liquid amount 50 µL. The resultant reaction liquid was incubated at 37°C for 1 hour.

A target DNA region (X, SEQ ID NO:1, region corresponding to base number 1142-1473 shown in Genbank Accession No. M80340) designed in LINE1 region that is known as human transposon exists plurally on human genomic DNA. As a specific oligonucleotide used for obtaining this, a 5'-end biotinylated oligonucleotide B1 comprising the nucleotide sequence of SEQ ID NO:4 binding with the target DNA region X by complementation was synthesized, and a 0.2 pmol/10 µL TE buffer solution was prepared. As a detection oligonucleotide binding with the target DNA region X by complementation for detecting the target DNA region X, a methylated oligonucleotide M1 comprising the nucleotide sequence of SEQ ID NO:5 to which the methylcytosine antibody is able to bind at 12 sites was synthesized, and 0.2 pmoL/10 µL TE buffer solution was prepared.

### <Target DNA region>

X:5'-
TAGAATATCCAATACAGAGAAGTGCTTAAAGGAGCTGATGGAGCTGAAAACCAAGGCTCGAG
AACTACGTGAAGAATGCAGAAGCCTCAGGAGCCGATGCGATCAACTGGAAGAAAGGGTATCA
GCAATGGAAGATGAAATGAATGAAATGAAGCGAGAAGGGAAGTTTAGAGAAAAAAGAATAAA
AAGAAATGAGCAAAGCCTCCAAGAAATATGGACTATGTGAAAAGACCAAATCTACGTCTGA
TTGGTGTACCTGAAAGTGATGTGGAGAATGGAACCAAGTTGGAAAACACTCTGCAGGATATT
ATCCAGGAGAACTTCCCCAATC -3' (SEQ ID NO:1)

<5'-end biotinylated oligonucleotide>
B1:5'-GGCTCCTGAGGCTTCTGCAT-3' (SEQ ID NO:4)
<Methylated oligonucleotide> N represents methylated cytosine.
M1:5'-TAAGCACTTCTCTGTATTGGATATNANANANANANANANANANANANA-3' (SEQ ID NO:5)

Thirty (30) µL of the reaction liquid of genomic DNA obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin, and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 1. In Solution A (genomic DNA derived from human blood 500 ng), Solution B (genomic DNA derived from human blood 50 ng), and Solution C (genomic DNA derived from human blood 5 ng) of genomic DNA derived from human blood, fluorescence intensity increased compared to Solution D (genomic DNA derived from human blood 0 ng: control solution), and the degree of increase was dependent on the concentration.

The present example revealed that the target DNA region is selected by the specific oligonucleotide and the detection oligonucleotide having sites to which the methylcytosine antibody is able to bind, so that a complex is formed, and by detecting and quantifying the complex, the target DNA region can be detected and quantified. Also it was revealed that by detecting and quantifying the target DNA region, genomic DNA can be detected and quantified.

### Example 2

The following TE buffer solutions were prepared in duplicate for each genomic DNA derived from human blood purchased from Clontech, Inc.
Solution A: genomic DNA derived from human blood 500 ng/20 µL TE buffer solution
Solution B: genomic DNA derived from human blood 50 ng/20 µL TE buffer solution
Solution C: genomic DNA derived from human blood 5 ng/20 µL TE buffer solution
Solution D: genomic DNA derived from human blood 0 ng/20 µL TE buffer solution (negative control solution)

Twenty (20) µL of each solution prepared above, 10U of restriction enzyme XspI, and 5 µL of 10x buffer optimum for XspI (200 mM Tris-HCl pH 8.5, 100 mM MgCl₂, 10 mM Dithiothreitol, 1000 mM KCl) were mixed, and added with sterilized ultrapure water to make the liquid amount 50 µL. The resultant reaction liquid was incubated at 37°C for 1 hour.

A target DNA region (Y, SEQ ID NO:2, region corresponding to base number 2692-2958 shown in Genbank Accession No. M80340) designed in LINE1 region that is known as human transposon exists plurally on human genomic DNA. As a specific oligonucleotide used for obtaining this, a 5'-end biotinylated oligonucleotide B2 comprising the nucleotide sequence of SEQ ID NO:6 binding with the target DNA region Y by complementation was synthesized, and a 0.2 pmol/10 µL TE buffer solution was prepared. As a detection oligonucleotide binding with the target DNA region Y by complementation for detecting the target DNA region Y, a methylated oligonucleotide M2 comprising the nucleotide sequence of SEQ ID NO:7 to which the methylcytosine antibody is able to bind at 12 sites was synthesized, and 0.2 pmoL/10 µL TE buffer solution was prepared.

### <Target DNA region>

Y:5′-
TAGAACTCAGGATTAAGAATCTCACTCAAAGCCGCTCAACTACATGGAAACTGAACAACCTG
CTCCTGAATGACTACTGGGTACATAACGAAATGAAGGCAGAAATAAAGATGTTCTTTGAAAC
CAACGAGAACAAAGACACCACATACCAGAATCTCTGGGACGCATTCAAAGCAGTGTGTAGAG
GGAAATTTATAGCACTAAATGCCTACAAGAGAAAGCAGGAAAGATCCAAAATTGACACCCTA
ACATCACAATTAAAAGAAC -3′ (SEQ ID NO:2)

<5'-end biotinylated oligonucleotide>
B2:5'- CCAGTAGTCATTCAGGAGCAG -3' (SEQ ID NO:6)
<Methylated oligonucleotide> N represents methylated cytosine.
M2:5'- TGAGTGAGATTCTTAATCCTGAGNANANANANANANANANANANANA -3' (SEQ ID NO:7)

Thirty (30) µL of the reaction liquid of genomic DNA obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin, and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 2. In Solution A (genomic DNA derived from human blood 500 ng), Solution B (genomic DNA derived from human blood 50 ng), and Solution C (genomic DNA derived from human blood 5 ng) of genomic DNA derived from human blood, fluorescence intensity increased compared to Solution D (genomic DNA derived from human blood 0 ng: control solution), and the degree of increase was dependent on the concentration.

The present example revealed that the target DNA region is selected by the specific oligonucleotide and the detection oligonucleotide having sites to which the methylcytosine antibody is able to bind, so that a complex is formed, and by detecting and quantifying the complex, the target DNA region can be detected and quantified. Also it was revealed that by detecting and quantifying the target DNA region, genomic DNA can be detected and quantified.

### Example 3

The following TE buffer solutions were prepared in duplicate for each genomic DNA derived from human blood purchased from Clontech, Inc.
Solution A: genomic DNA derived from human blood 500 ng/20 µL TE buffer solution
Solution B: genomic DNA derived from human blood 50 ng/20 µL TE buffer solution
Solution C: genomic DNA derived from human blood 5 ng/20 µL TE buffer solution
Solution D: genomic DNA derived from human blood 0 ng/20 µL TE buffer solution (negative control solution)

Twenty (20) µL of each solution prepared above, 10U of restriction enzyme XspI, and 5 µL of 10x buffer optimum for XspI (200 mM Tris-HCl pH 8.5, 100 mM MgCl₂, 10 mM Dithiothreitol, 1000 mM KCl) were mixed, and added with sterilized ultrapure water to make the liquid amount 50 µL. The resultant reaction liquid was incubated at 37°C for 1 hour.

A target DNA region (X, SEQ ID NO:1, region corresponding to base number 1142-1473 shown in Genbank Accession No. M80340) designed in LINE1 region that is known as human transposon exists plurally on human genomic DNA. As a specific oligonucleotide used for obtaining this, a 5'-end biotinylated oligonucleotide B3 comprising the nucleotide sequence of SEQ ID NO:8 binding with the target DNA region X by complementation was synthesized, and a 0.2 pmol/10 µL TE buffer solution was prepared. As a detection oligonucleotide binding with the target DNA region X by complementation for detecting the target DNA region X, a methylated oligonucleotide M1 comprising the nucleotide sequence of SEQ ID NO:5 to which the methylcytosine antibody is able to bind at 12 sites was synthesized, and 0.2 pmoL/10 µL TE buffer solution was prepared.

### <Target DNA region>

X:5'-

TAGAATATCCAATACAGAGAAGTGCTTAAAGGAGCTGATGGAGCTGAAAACCAAGGCTCGAG
AACTACGTGAAGAATGCAGAAGCCTCAGGAGCCGATGCGATCAACTGGAAGAAAGGGTATCA
GCAATGGAAGATGAAATGAATGAAATGAAGCGAGAAGGGAAGTTTAGAGAAAAAAGAATAAA
AAGAAATGAGCAAAGCCTCCAAGAAATATGGGACTATGTGAAAAGACCAAATCTACGTCTGA
TTGGTGTACCTGAAAGTGATGTGGAGAATGGAACCAAGTTGGAAAACACTCTGCAGGATATT
ATCCAGGAGAACTTCCCCAATC -3′ (SEQ ID NO:1)

<5'-end biotinylated oligonucleotide>
B3:5' - TCAGGTACACCAATCAGACGTA -3' (SEQ ID NO:8)
<Methylated oligonucleotide> N represents methylated cytosine.
M1:5' - TAAGCACTTCTCTGTATTGGATATNANANANANANANANANANANANA -3' (SEQ ID NO:5)

Thirty (30) µL of the reaction liquid of genomic DNA obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin, and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂ PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 3. In Solution A (genomic DNA derived from human blood 500 ng), Solution B (genomic DNA derived from human blood 50 ng), and Solution C (genomic DNA derived from human blood 5 ng) of genomic DNA derived from human blood, fluorescence intensity increased compared to Solution D (genomic DNA derived from human blood 0 ng: control solution), and the degree of increase was dependent on the concentration.

The present example revealed that the target DNA region is selected by the specific oligonucleotide and the detection oligonucleotide having sites to which the methylcytosine antibody is able to bind, so that a complex is formed, and by detecting and quantifying the complex, the target DNA region can be detected and quantified. Also it was revealed that by detecting and quantifying the target DNA region, genomic DNA can be detected and quantified.

### Example 4

The following TE buffer solutions were prepared in duplicate for each genomic DNA derived from human blood purchased from Clontech, Inc.
Solution A: genomic DNA derived from human blood 500 ng/20 µL TE buffer solution
Solution B: genomic DNA derived from human blood 50 ng/20 µL TE buffer solution
Solution C: genomic DNA derived from human blood 5 ng/20 µL TE buffer solution
Solution D: genomic DNA derived from human blood 0 ng/20 µL TE buffer solution (negative control solution)

Twenty (20) µL of each solution prepared above, 10U of restriction enzyme XspI, and 5 µL of 10x buffer optimum for XspI (200 mM Tris-HCl pH 8.5, 100 mM MgCl₂, 10 mM Dithiothreitol, 1000 mM KCl) were mixed, and added with sterilized ultrapure water to make the liquid amount 50 µL. The resultant reaction liquid was incubated at 37°C for 1 hour.

A target DNA region (X, SEQ ID NO:1, region corresponding to base number 1142-1473 shown in Genbank Accession No. M80340) designed in LINE1 region that is known as human transposon exists plurally on human genomic DNA. As a specific oligonucleotide used for obtaining this, a 5'-end biotinylated oligonucleotide B3 comprising the nucleotide sequence of SEQ ID NO:8 binding with the target DNA region X by complementation was synthesized, and a 0.2 pmol/10 µL TE buffer solution was prepared. As a detection oligonucleotide binding with the target DNA region X by complementation for detecting the target DNA region X, a methylated oligonucleotide M3 comprising the nucleotide sequence of SEQ ID NO:9 to which the methylcytosine antibody is able to bind at 12 sites was synthesized, and 0.2 pmoL/10 µL TE buffer solution was prepared.

### <Target DNA region>

X:5′-
TAGAATATCCAATACAGAGAAGTGCTTAAAGGAGCTGATGGAGCTGAAAACCAAGGCTCGAG
AACTACGTGAAGAATGCAGAAGCCTCAGGAGCCGATGCGATCAACTGGAAGAAAGGGTATCA
GCAATGGAAGATGAAATGAATGAAATGAAGCGAGAAGGGAAGTTTAGAGAAAAAAGAATAAA
AAGAAATGAGCAAAGCCTCCAAGAAATATGGGACTATGTGAAAAGACCAAATCTACGTCTGA
TTGGTGTACCTGAAAGTGATGTGGAGAATGGAACCAAGTTGGAAAACACTCTGCAGGATATT
ATCCAGGAGAACTTCCCCAATC -3′ (SEQ ID NO:1)

<5'-end biotinylated oligonucleotide>
B3:5' - TCAGGTACACCAATCAGACGTA -3' (SEQ ID NO:8)
<Methylated oligonucleotide> N represents methylated cytosine.
M3:5' - ATCGGCTCCTGAGGCTTCTGNANANANANANANANANANANANA -3' (SEQ ID NO:9)

Thirty (30) µL of the reaction liquid of genomic DNA obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin, and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂ PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 4. In Solution A (genomic DNA derived from human blood 500 ng), Solution B (genomic DNA derived from human blood 50 ng), and Solution C (genomic DNA derived from human blood 5 ng) of genomic DNA derived from human blood, fluorescence intensity increased compared to Solution D (genomic DNA derived from human blood 0 ng: control solution), and the degree of increase was dependent on the concentration.

The present example revealed that the target DNA region is selected by the specific oligonucleotide and the detection oligonucleotide having sites to which the methylcytosine antibody is able to bind, so that a complex is formed, and by detecting and quantifying the complex, the target DNA region can be detected and quantified. Also it was revealed that by detecting and quantifying the target DNA region, genomic DNA can be detected and quantified.

### Example 5

The following TE buffer solutions were prepared in duplicate for each genomic DNA derived from human blood purchased from Clontech, Inc.
Solution A: genomic DNA derived from human blood 500 ng/20 µL TE buffer solution
Solution B: genomic DNA derived from human blood 50 ng/20 µL TE buffer solution
Solution C: genomic DNA derived from human blood 5 ng/20 µL TE buffer solution
Solution D: genomic DNA derived from human blood 0 ng/20 µL TE buffer solution (negative control solution)

Twenty (20) µL of each solution prepared above, 10U of restriction enzyme XspI, and 5 µL of 10x buffer optimum for XspI (200 mM Tris-HCl pH 8.5, 100 mM MgCl₂, 10 mM Dithiothreitol, 1000 mM KCl) were mixed, and added with sterilized ultrapure water to make the liquid amount 50 µL. The resultant reaction liquid was incubated at 37°C for 1 hour.

A target DNA region (X, SEQ ID NO:1, region corresponding to base number 1142-1473 shown in Genbank Accession No. M80340) designed in LINE1 region that is known as human transposon exists plurally on human genomic DNA. As a specific oligonucleotide used for obtaining this, a 5'-end biotinylated oligonucleotide B3 comprising the nucleotide sequence of SEQ ID NO:8 binding with the target DNA region X by complementation was synthesized, and a 0.2 pmol/10 µL TE buffer solution was prepared. As a detection oligonucleotide binding with the target DNA region X by complementation for detecting the target DNA region X, a methylated oligonucleotide M1 comprising the nucleotide sequence of SEQ ID NO:5 to which the methylcytosine antibody is able to bind at 12 sites was synthesized, and as a detection oligonucleotide binding with the target DNA region X by complementation, a methylated oligonucleotide M3 comprising the nucleotide sequence of SEQ ID NO:9 to which the methylcytosine antibody is able to bind at 12 sites was synthesized and 0.2 pmoL of respective detection oligonucleotides/10 µL TE buffer solution was prepared.

### <Target DNA region>

X:5'-

TAGAATATCCAATACAGAGAAGTGCTTAAAGGAGCTGATGGAGCTGAAAACCAAGGCTCGAG

AACTACGTGAAGAATGCAGAAGCCTCAGGAGCCGATGCGATCAACTGGAAGAAAGGGTATCA

GCAATGGAAGATGAAATGAATGAAATGAAGCGAGAAGGGAAGTTTAGAGAAAAAAGAATAAA

AAGAAATGAGCAAAGCCTCCAAGAAATATGGGACTATGTGAAAAGACCAAATCTACGTCTGA
TTGGTGTACCTGAAAGTGATGTGGAGAATGGAACCAAGTTGGAAAACACTCTGCAGGATATT
ATCCAGGAGAACTTCCCCAATC -3′ (SEQ ID NO:1)

<5'-end biotinylated oligonucleotide>
B3:5' - TCAGGTACACCAATCAGACGTA -3' (SEQ ID NO:8)
<Methylated oligonucleotide> N represents methylated cytosine.
M1:5' - TAAGCACTTCTCTGTATTGGATATNANANANANANANANANANANANA -3' (SEQ ID NO:5)
<Methylated oligonucleotide> N represents methylated cytosine.
M3:5' - ATCGGCTCCTGAGGCTTCTGNANANANANANANANANANANANA -3' (SEQ ID NO:9)

Thirty (30) µL of the reaction liquid obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin, and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 5. In Solution A (genomic DNA derived from human blood 500 ng), Solution B (genomic DNA derived from human blood 50 ng), and Solution C (genomic DNA derived from human blood 5 ng) of genomic DNA derived from human blood, fluorescence intensity increased compared to Solution D (genomic DNA derived from human blood 0 ng: control solution), and the degree of increase was dependent on the concentration.

The present example revealed that the target DNA region is selected by the specific oligonucleotide and the detection oligonucleotide having sites to which the methylcytosine antibody is able to bind, so that a complex is formed, and by detecting and quantifying the complex, the target DNA region can be detected and quantified. Also it was revealed that by detecting and quantifying the target DNA region, genomic DNA can be detected and quantified.

### Example 6

The following TE buffer solutions were prepared in duplicate for each genomic DNA derived from human blood purchased from Clontech, Inc.
Solution A: genomic DNA derived from human blood 500 ng/20 µL TE buffer solution
Solution B: genomic DNA derived from human blood 50 ng/20 µL TE buffer solution
Solution C: genomic DNA derived from human blood 5 ng/20 µL TE buffer solution
Solution D: genomic DNA derived from human blood 0 ng/20 µL TE buffer solution (negative control solution)

Twenty (20) µL of each solution prepared above, 10U of restriction enzyme XspI, and 5 µL of 10x buffer optimum for XspI (200 mM Tris-HCl pH 8.5, 100 mM MgCl₂, 10 mM Dithiothreitol, 1000 mM KC1) were mixed, and added with sterilized ultrapure water to make the liquid amount 50 µL. The resultant reaction liquid was incubated at 37°C for 1 hour.

A target DNA region (Z, SEQ ID NO:3, region corresponding to base number 178-262 shown in Genbank Accession No. AF458110) designed in Alu region that is known as human transposon exists plurally on human genomic DNA. As a specific oligonucleotide used for obtaining this, a 5'-end biotinylated oligonucleotide B4 comprising the nucleotide sequence of SEQ ID NO:10 binding with the target DNA region Z by complementation was synthesized, and a 0.2 pmol/10 µL TE buffer solution was prepared. As a detection oligonucleotide binding with the target DNA region Z by complementation for detecting the target DNA region Z, a methylated oligonucleotide M4 comprising the nucleotide sequence of SEQ ID NO:11 to which the methylcytosine antibody is able to bind at 12 sites was synthesized and 0.2 pmoL /10 µL TE buffer solution was prepared.

### <Target DNA region>

Z:5' -

CGGGCGCGGTGGCTCACGCCTGTAATCCCAGCACTTTGGGAGGCCGAGGTGGGCGGATCACG

AGGTCAGGAGATCGAGACCATCC -3' (SEQ ID NO:3)

<5'-end biotinylated oligonucleotide>
B4:5' - GGATGGTCTCGATCTCCTGAC -3' (SEQ ID NO:10)
<Methylated oligonucleotide> N represents methylated cytosine.
M4:5' - GATTACAGGCGTGAGCCACCNANANANANANANANANANANANA -3' (SEQ ID NO:11)

Thirty (30) µL of the reaction liquid obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin, and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 6. In Solution A (genomic DNA derived from human blood 500 ng), Solution B (genomic DNA derived from human blood 50 ng), and Solution C (genomic DNA derived from human blood 5 ng) of genomic DNA derived from human blood, fluorescence intensity increased compared to Solution D (genomic DNA derived from human blood 0 ng: control solution), and the degree of increase was dependent on the concentration.

The present example revealed that the target DNA region is selected by the specific oligonucleotide and the detection oligonucleotide having sites to which the methylcytosine antibody is able to bind, so that a complex is formed, and by detecting and quantifying the complex, the target DNA region can be detected and quantified. Also it was revealed that by detecting and quantifying the target DNA region, genomic DNA can be detected and quantified.

### Example 7

As a serum sample, a mixture of a TE buffer solution of genomic DNA derived from human blood (Human Genomic DNA, #636401, available from Clontech, Inc.) and a serum collected from a rat (Wistar Hannover) was prepared in quadruplet for each.
Serum sample A: genomic DNA derived from human blood 100 ng/10 µL TE buffer solution + rat serum 10 µL
Serum sample B: genomic DNA derived from human blood 10 ng/10 µL TE buffer solution + rat serum 10 µL
Serum sample C: genomic DNA derived from human blood 1 ng/10 µL TE buffer solution + rat serum 10 µL
Serum sample D: genomic DNA derived from human blood 0 ng/10 µL TE buffer solution + rat serum 10 µL (negative control solution)

For Serum samples A to D prepared in the above, the following Treatment 1 or Treatment 2 was conducted in duplicate for each.

### Treatment 1:

Twenty (20) µL of a serum sample, 4 µL of a buffer (500 mM Tris-HCl (pH 7.5), and 100 mM MgCl₂, 10 mM DTT, 1000 mM NaCl) were mixed and the resultant mixture was added with sterilized ultrapure water to make the liquid amount 40 µL, and mixed. Thereafter, the PCR tube was kept at 95°C for 10 minutes, and kept at 4°C for 10 minutes, and then returned to room temperature. After centrifugation at 9100g for 10 minutes, the supernatant was collected.

### Treatment 2:

Twenty (20) µL of a serum sample, 4 µL of a buffer (330 mM Tris-Acetate (pH 7.9), and 100 mM Mg(OAc)₂, 5 mM DTT, 660 mM KOAc) were mixed and the resultant mixture was added with sterilized ultrapure water to make the liquid amount 40 µL, and mixed. Thereafter, the PCR tube was kept at 95°C for 10 minutes, and kept at 4°C for 10 minutes, and then returned to room temperature. After centrifugation at 9100g for 10 minutes, the supernatant was collected.

Twenty (20) µL of each solution prepared by the above Treatment 1 or Treatment 2, 4U of restriction enzyme MspI, and 5 µL of 10x buffer optimum for MspI (100 mM Tris-HCl pH 7.5, 100 mM MgCl₂, 10 mM Dithiothreitol, 500 mM NaCl) were mixed, and added with sterilized ultrapure water to prepare a reaction liquid having a liquid amount of 50 µL. The reaction liquid was incubated at 37°C for 1 hour.

As a specific oligonucleotide used for obtaining a target DNA region (Z, SEQ ID NO:3, region corresponding to base number 178-262 shown in Genbank Accession No. AF458110) comprising the nucleotide sequence of SEQ ID NO:3 designed in Alu region that is known as human transposon, a 5'-end biotin-labeled oligonucleotide B4 comprising the nucleotide sequence of SEQ ID NO:10 that binds with the target DNA region Z by complementation was synthesized, and a 0.2 pmoL/10 µL TE buffer solution was prepared.

### <Target DNA region>

Z:5'-
CGGGCGCGGTGGCTCACGCCTGTAATCCCAGCACTTTGGGAGGCCGAGGTGGGCGGATCACG
AGGTCAGGAGATCGAGACCATCC -3' (SEQ ID NO:3)

<5'-end biotinylated oligonucleotide>
B4:5'- GGATGGTCTCGATCTCCTGAC -3' (SEQ ID NO:10)

As a detection oligonucleotide binding with the target DNA region Z by complementation for detecting the target DNA region Z, a methylated oligonucleotide M4 comprising the nucleotide sequence of SEQ ID NO:11 to which the methylcytosine antibody is able to bind at 12 sites was synthesized and 0.2 pmoL /10 µL TE buffer solution was prepared.
<Methylated-oligonucleotide> N represents methylated cytosine.
M4:5'- GATTACAGGCGTGAGCCACCNANANANANANANANANANANANA -3' (SEQ ID NO:11)

Thirty (30) µL of the reaction liquid obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin (StreptaWell, #11645692001, Roche), and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KHz PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 7 and Fig. 8. In Treatment 1, fluorescence intensity increased in a concentration dependent manner in Solution A (genomic DNA derived from human blood 100 ng), Solution B (genomic DNA derived from human blood 10 ng), and Solution C (genomic DNA derived from human blood 1 ng) of genomic DNA derived from human blood, compared to Solution D (genomic DNA derived from human blood 0 ng:control solution) (Fig. 7). On the other hand, in Treatment 2, fluorescence intensity increased in Solution A (genomic DNA derived from human blood 100 ng) of genomic DNA derived from human blood, compared to Solution D (genomic DNA derived from human blood 0 ng:control solution), however, increase in fluorescence intensity was not observed in Solution B (genomic DNA derived from human blood 10 ng) and Solution C (genomic DNA derived from human blood 1 ng).

The present example revealed that by forming and selecting the complex of the methylcytosine antibody, the methylated oligonucleotide, and the 5'-end biotin-labeled oligonucleotide, and detecting the methylcytosine antibody in the complex by its function, human genomic DNA in serum can be detected and quantifying with excellent sensitivity. In Treatment 1, human genomic DNA in serum was detected with better sensitivity compared to in Treatment 2.

### Example 8

As a serum sample, a mixture of a TE buffer solution of genomic DNA derived from human blood (Human Genomic DNA, #636401, available from Clontech, Inc.) and Human serums purchased from Kohjin Bio Co., Ltd (individual human serums) was prepared in quadruplet for each.
Serum sample A: genomic DNA derived from human blood 10 ng/10 µL TE buffer solution + human serum 40 µL
Serum sample B: genomic DNA derived from human blood 1 ng/10 µL TE buffer solution + human serum 40 µL
Serum sample C: genomic DNA derived from human blood 0 ng/10 µL TE buffer solution + human serum 40 µL (negative control solution)

For Serum samples A to C prepared in the above, the following Treatment 1 or Treatment 2 was conducted in duplicate for each.

### Treatment 1:

Fifty (50) µL of a serum sample, 20 µL of a buffer (500 mM Tris-HCl (pH 7.5), and 100 mM MgCl₂, 10 mM DTT, 1000 mM NaCl) were mixed and the resultant mixture was added with sterilized ultrapure water to make the liquid amount 100 µL, and mixed. Thereafter, it was kept at 95°C for 10 minutes, and kept at 4°C for 10 minutes, and then returned to room temperature. After centrifugation at 9100g for 10 minutes, the supernatant was collected.

### Treatment 2:

Fifty (50) µL of a serum sample, 10 µL of a buffer (500 mM Tris-HCl (pH 7.5), and 100 mM MgCl₂, 10 mM DTT, 1000 mM NaCl) were mixed and the resultant mixture was added with sterilized ultrapure water to make the liquid amount 100 µL, and mixed. Thereafter, it was kept at 95°C for 10 minutes, and kept at 4°C for 10 minutes, and then returned to room temperature. After centrifugation at 9100g for 10 minutes, the supernatant was collected.

Twenty (20) µL of each solution prepared by the above Treatment 1 or Treatment 2, 2U of restriction enzyme MspI, and 5 µL of 10x buffer optimum for MspI (100 mM Tris-HCl pH 7.5, 100 mM MgCl₂, 10 mM Dithiothreitol, 500 mM NaCl) were mixed, and added with sterilized ultrapure water to prepare a reaction liquid having a liquid amount of 50 µL. The reaction liquid was incubated at 37°C for 1 hour.

As a specific oligonucleotide used for obtaining a target DNA region (Z, SEQ ID NO:3, region corresponding to base number 178-262 shown in Genbank Accession No. AF458110) comprising the nucleotide sequence of SEQ ID NO:3 designed in Alu region that is known as human transposon, a 5'-end biotin-labeled oligonucleotide B4 comprising the nucleotide sequence of SEQ ID NO:10 that binds with the target DNA region Z by complementation was synthesized, and a 0.2 pmoL/10 µL TE buffer solution was prepared.

### <Target DNA region>

Z:5'-
CGGGCGCGGTGGCTCACGCCTGTAATCCCAGCACTTTGGGAGGCCGAGGTGGGCGGATCACG
AGGTCAGGAGATCGAGACCATCC -3' (SEQ ID NO:3)

<5'-end biotinylated oligonucleotide>
B4:5'- GGATGGTCTCGATCTCCTGAC -3' (SEQ ID NO:10)

As a detection oligonucleotide binding with the target DNA region Z by complementation for detecting the target DNA region Z, a methylated oligonucleotide M4 comprising the nucleotide sequence of SEQ ID NO:11 to which the methylcytosine antibody is able to bind at 12 smites was synthesized and 0.2 pmoL /10 µL TE buffer solution was prepared.
<Methylated oligonucleotide> N represents methylated cytosine.
M4:5'- GATTACAGGCGTGAGCCACCNANANANANANANANANANANANA -3' (SEQ ID NO:11)

Thirty (30) µL of the reaction liquid obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin (StreptaWell, #11645692001, Roche), and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

The result is shown in Fig. 9 and Fig. 10. In Treatment 1 and Treatment 2, fluorescence intensity increased in a concentration dependent manner in Solution A (genomic DNA derived from human blood 10 ng), and Solution B (genomic DNA derived from human blood 1 ng) of genomic DNA derived from human blood, compared to Solution C (genomic DNA derived from human blood 0 ng:control solution).

The present example revealed that by forming and selecting the complex of the methylcytosine antibody, the methylated oligonucleotide, and the 5'-end biotin-labeled oligonucleotide, and detecting the methylcytosine antibody in the complex by its function, human genomic DNA in serum can be detected and quantifying with excellent sensitivity. In Treatment 1, human genomic DNA in serum was detected with better sensitivity compared to in Treatment 2.

### Example 9

As a serum sample, the following human serums were used. Human serums purchased from Kohjin Bio Co., Ltd (individual human serums)

### Lot No.:

N51438 (healthy subject)
N51439 (healthy subject)
N51441 (healthy subject)
Human serums purchased from ProMedDx (individual human serums) Lot No.:
11171268 (healthy subject, age 56, male)
11171292 (healthy subject, age 62, male)
11171297 (healthy subject, age 67, male)
11171314 (healthy subject, age 75, male)
11171327 (healthy subject, age 70, male)
11202510 (healthy subject, age 67, female)
11202522 (healthy subject, age 64, female)
11202527 (healthy subject, age 52, female)
11202615 (healthy subject, age 75, female)
11202618 (healthy subject, age 78, female)
10958886 (healthy subject, age 56, male)
10958979 (healthy subject, age 39, male)
10958980 (healthy subject, age 45, male)
10960268 (healthy subject, age 37, male)
10960272 (healthy subject, age 50, male)
10960276 (healthy subject, age 30, male)
10960285 (healthy subject, age 39, male)
11003457 (healthy subject, age 38, male)
11003479 (healthy subject, age 51, male)
11003480 (healthy subject, age 48, male)
11324997 (healthy subject, age 59, male)
11325001 (healthy subject, age 61, male)
10325022 (healthy subject, age 61, male)
11325032 (healthy subject, age 60, male)
11325062 (healthy subject, age 69, male)
10870623 (breast cancer patient, age 33, female)
10929521 (breast cancer patient, age 55, female)
10989644 (breast cancer patient, age 45, female)
11209430 (breast cancer patient, age 80, female)
10929514 (breast cancer patient, age 57, female)
10843055 (breast cancer patient, age 59, female)
10984680 (breast cancer patient, age 64, female)
11209428 (breast cancer patient, age 55, female)
10840414 (lung cancer patient, age 54, female)
10929506 (lung cancer patient, age 55, male)
11091955 (lung cancer patient, age 76, female)
11103346 (lung cancer patient, age 66, female)
11142322 (lung cancer patient, age 62, female)
11152564 (lung cancer patient, age 67, male)
11152571 (lung cancer patient, age 67, male)
11153198 (lung cancer patient, age 69, female)
11209435 (lung cancer patient, age 61, male)
11230621 (lung cancer patient, age 71, female)
11153192 (lung cancer patient, age 59, male)
10715942 (lung cancer patient, age 64, male)
10840422 (lung cancer patient, age 78, female)
10935547 (prostate cancer patient, age 83, male)
11000243 (prostate cancer patient, age 78, male)
11071226 (prostate cancer patient, age 84, male)

For each of the above serum samples, the following treatment was conducted.

Twenty (20) µL of a serum sample, 4 µL of a buffer (500 mM Tris-HCl (pH 7.5), and 100 mM MgCl₂, 10 mM DTT, 1000 mM NaCl) were mixed and the resultant mixture was added with sterilized ultrapure water to make the liquid amount 40 µL, and mixed. Thereafter, the PCR tube was kept at 95°C for 10 minutes, and kept at 4°C for 10 minutes, and then returned to room temperature. After centrifugation at 9100g for 10 minutes, the supernatant was collected.

Twenty (20) µL of each solution prepared by the above treatment, 2U of restriction enzyme MspI, and 5 µL of 10x buffer optimum for MspI (100 mM Tris-HCl pH 7.5, 100 mM MgCl₂, 10 mM Dithiothreitol, 500 mM NaCl) were mixed, and added with sterilized ultrapure water to prepare a reaction liquid having a liquid amount of 50 µL. The reaction liquid was incubated at 37°C for 1 hour.

As a specific oligonucleotide used for obtaining a target DNA region (Z, SEQ ID NO:3, region corresponding to base number 178-262 shown in Genbank Accession No. AF458110) comprising the nucleotide sequence of SEQ ID NO:3 designed in Alu region that is known as human transposon, a 5'-end biotin-labeled oligonucleotide B4 comprising the nucleotide sequence of SEQ ID NO:10 that binds with the target DNA region Z by complementation was synthesized, and a 0.2 pmoL/10 µL TE buffer solution was prepared.

### <Target DNA region>

Z:5'-

CGGGCGCGGTGGCTCACGCCTGTAATCCCAGCACTTTGGGAGGCCGAGGTGGGCGGATCACG

AGGTCAGGAGATCGAGACCATCC -3' (SEQ ID NO:3)

<5'-end biotinylated oligonucleotide>
B4:5'- GGATGGTCTCGATCTCCTGAC -3' (SEQ ID NO:10)

As a detection oligonucleotide binding with the target DNA region Z by complementation for detecting the target DNA region Z, a methylated oligonucleotide M4 comprising the nucleotide sequence of SEQ ID NO:11 to which the methylcytosine antibody is able to bind at 12 sites was synthesized and 0.2 pmoL /10 µL TE buffer solution was prepared.
<Methylated oligonucleotide> N represents methylated cytosine.
M4:5'- GATTACAGGCGTGAGCCACCNANANANANANANANANANANANA -3' (SEQ ID NO:11)

Thirty (30) µL of the reaction liquid obtained in the above, 10 µL of the specific oligonucleotide solution, 10 µL of the detection oligonucleotide solution, 10 µL of a buffer (330 mM Tris-Acetate pH 7.9, 660 mM KOAc, 100 mM MgOAc₂, 5 mM Dithiothreitol), 10 µL of 100 mM MgCl₂ solution, and 10 µL of 1 mg/mL BSA solution were added, and the resultant mixture was added with sterilized ultrapure water to make the liquid amount 100 µL and mixed. Thereafter, for forming a double strand of the target DNA region and the specific oligonucleotide, and for concurrently forming a double strand of the target DNA region and the detection oligonucleotide (namely, for forming a detection complex comprising the target DNA region, the specific oligonucleotide, and the detection oligonucleotide), the above mixture was heated at 95°C for 10 minutes, rapidly cooled to 70°C, and retained at this temperature for 10 minutes. Then the reaction was cooled to 50°C and retained for 10 minutes, and further retained at 37°C for 10 minutes, and then returned to room temperature.

One hundred (100) µL of the obtained reaction liquid was transferred to a 8-well strip coated with streptavidin (StreptaWell, #11645692001, Roche), and left still for about 30 minutes at room temperature, and thus a detection complex comprising the target DNA region, the specific oligonucleotide and the detection oligonucleotide was immobilized to the 8-well strip via a biotin-streptavidin bond. Thereafter, the solution was removed by decantation, and each well was washed three times with 200 µL of a washing buffer [0.05% Tween20-containing phosphate buffer (1 mM KH₂ PO₄, 3 mM Na₂HPO₄ 7H₂O, 154 mM NaCl pH7.4)].

Each well was added with 100 µL of a methylcytosine antibody [available from Aviva Systems Biology, Inc., 0.5 µg/mL 0.1% BSA-containing phosphate buffer (1 mM KH₂PO₄, 3 mM Na₂HPO 7H₂O, 154 mM NaCl pH7.4) solution], and left still for 1 hour at room temperature. Thereafter, the solution was removed by decantation, and each well was washed three times with 200 µL of a washing buffer [0.05% Tween20-containing phosphate buffer (1 mM KHZ PO₄, 3 mM Na₂HPO₄ 7H₂O, 154 mM NaCl pH7.4)].

Thereafter, 100 µL of an Eu-N1-labeled mouse IgG antibody [available from PerkinElmer, Inc., 0.05 µg/mL 0.1% BSA-containing phosphate buffer (1 mM KH₂PO₄, 3 mM Na₂HPO 7H₂O, 154 mM NaCl pH7.4) solution] was added to each well, and left still for 1 hour at room temperature. After leaving still, the solution was removed by decantation, and each well was washed three times with 200 µL of a washing buffer [0.05% Tween20-containing phosphate buffer (1 mM KH₂PO₄, 3 mM Na₂HPO₄ 7H₂O, 154 mM NaCl pH7.4)].

Each well was added with 150 µL of Enhancement Solution (available from PerkinElmer, Inc.), and mixed, and shaken for about 5 minutes at room temperature. Thereafter, fluorescence was measured at excitation 340 nm/fluorescence 612 nm.

DNA in the solution obtained by the above enzyme treatment (MspI treatment) was quantified by real-time PCR.

As a standard sample for measuring concentration, a MspI-treated human genomic DNA solution was prepared in the following manner. A 5 ng/µL TE buffer solution of genomic DNA derived from human blood (Human Genomic DNA, #636401, Clontech) was prepared, and 20 µL of the solution, 2 U of restriction enzyme MspI, and 5 µL of a 10xbuffer optimum for MspI (100 mM Tris-HCl pH 7.5, 100 mM MgCl₂, 10 mM Dithiothreitol, 500 mM NaCl) were mixed, and added with sterilized ultrapure water to prepare a reaction liquid having a liquid amount of 50 µL. The reaction liquid was incubated at 37°C for 1 hour. For the obtained reaction liquid, 10⁻⁵, 10-⁴, 10⁻³, 10⁻², 10⁻¹, 1, 10 ng/5 µL solutions were prepared by dilution with TE buffer.

For amplifying a target DNA region (Z, SEQ ID NO:3, region corresponding to the nucleotide number 178-262 shown in Genbank Accession No. AF458110) designed in Alu region known as human transposon and quantifying by real-time PCR, a forward primer (F) and a reverse primer (R) were designed.

### <Target DNA region>

Z:5'-
CGGGCGCGGTGGCTCACGCCTGTAATCCCAGCACTTTGGGAGGCCGAGGTGGGCGGATCACG
AGGTCAGGAGATCGAGACCATCC -3' (SEQ ID NO:3)

<Forward primer>
F:5'- GGTGGCTCACGCCTGTAATC -3' (SEQ ID NO:16)
<Reverse primer>
R:5'- GGATGGTCTCGATCTCCTGAC -3' (SEQ ID NO:17)

A reaction liquid of PCR was prepared by mixing 5 µL of the MspI-treated human genomic DNA solution prepared in the above or the standard sample for measuring concentration prepared in the above serving as a template, each 1.5 µL of 5 µM solutions of primers each comprising the nucleotide sequence of SEQ ID NO:16 and the nucleotide sequence of SEQ ID NO:17, 0.1x amount of SYBR® Green I (Lonza), 2.5 µL of each 2 mM dNTP, 2.5 µL of a 10xPCR buffer (100 mM Tris-HCl pH 8.3, 500 mM KC1, 15 mM MgCl₂, 0.01% Gelatin), and 0.125 µL of thermostable DNA polymerase (AmpliTaq Gold, 5 U/µL, ABI), and adding sterilized ultrapure water to make the liquid amount 25 µL. Real-time PCR was conducted using Mx3005P (Stratagene). After retaining the reaction liquid at 95°C for 10 minutes, PCR was conducted by 40 cycles each consisting of 30 seconds at 95°C, 30 seconds at 61°C and 45 seconds at 72°C, to amplify the target DNA region. According to a result of the real-time PCR, DNA in a serum sample was quantified.

The results are shown in Fig. 11 and Fig. 12. A measured value by the present method, and a value quantified by the real-time PCR were compared, to reveal that there is a correlation (coefficient of correlation: R = 0.88)(Fig. 11). Further, the results quantified for human serum samples aged 59 or younger were compared between cancer patients and healty subjects, to reveal that serum DNA concentration increases in cancer patients (Fig. 12).

### INDUSTRIAL APPLICABILITY

According to the present invention, it becomes possible to provide a method for quantifying or detecting DNA having a target DNA region in a simple and convenient manner.
Free Text in Sequence Listing
SEQ ID NO:4
Designed biotinated oligonucleotide for fixation
SEQ ID NO:5
Designed oligonucleotide
SEQ ID NO:6
Designed biotinated oligonucleotide for fixation
SEQ ID NO:7
Designed oligonucleotide
SEQ ID NO:8
Designed biotinated oligonucleotide for fixation
SEQ ID NO:9
Designed oligonucleotide
SEQ ID NO:10
Designed biotinated oligonucleotide for fixation
SEQ ID NO:11
Designed oligonucleotide
SEQ ID NO:12
Designed oligonucleotide
SEQ ID NO:13
Designed oligonucleotide
SEQ ID N0:14
Designed oligonucleotide
SEQ ID NO:15
Designed oligonucleotide
SEQ ID NO:16
Designed oligonucleotide for amplification
SEQ ID NO:17
Designed oligonucleotide for amplification

## Claims

1. A method for quantifying or detecting DNA which comprises a target DNA region existing plurally in genome and is contained in a specimen, comprising:
(1) First step of preparing a specimen containing a test oligonucleotide which is DNA comprising a target DNA region existing plurally in genome,
(2) Second step of mixing the test oligonucleotide contained in the specimen prepared in First step, a detection oligonucleotide capable of complementarily binding with the test oligonucleotide, a specific oligonucleotide capable of complementarily binding with the test oligonucleotide and a support capable of binding with the specific oligonucleotide, to form a detection complex comprising the test oligonucleotide, the detection oligonucleotide, the specific oligonucleotide and the support, and
(3) Third step of quantifying or detecting said DNA comprising the target DNA region by detecting the detection oligonucleotide contained in the detection complex formed in Second step by its identification function.

2. The method according to claim 1, wherein the detection oligonucleotide is a composite detection oligonucleotide comprising a plurality of oligonucleotides containing a plurality of methylation sites.

3. The method according to claim 1 or 2, wherein the identification function of the detection oligonucleotide is an identification function of a methylated DNA antibody that binds with methylated DNA of the detection oligonucleotide.

4. The method according to any one of claims 1 to 3,
wherein the DNA comprising the target DNA region existing plurally in genome comprises a nucleotide sequence constituting a repetition sequence in genome or a part thereof.

5. The method according to any one of claims 1 to 3,
wherein the DNA comprising the target DNA region existing plurally in genome is DNA comprising a nucleotide sequence of a duplicate gene or a pseudo gene in genome, or a part thereof.

6. The method according to any one of claims 1 to 3,
wherein the DNA comprising the target DNA region existing plurally in genome is DNA comprising a nucleotide sequence classified into a LINE or an Alu sequence.

7. The method according to claim 4, wherein the nucleotide sequence constituting the repetitive sequence in genome comprises any of the following nucleotide sequences:
(1) the nucleotide sequence of SEQ ID NO:1, or a nucleotide sequence having 80% or more sequence identity to the same;
(2) the nucleotide sequence of SEQ ID NO:2, or a nucleotide sequence having 80% or more sequence identity to the same; and
(3) the nucleotide sequence of SEQ ID NO:3, or a nucleotide sequence having 80% or more sequence identity to the same.

8. The method according to any one of claims 1 to 7, wherein the detection oligonucleotide comprises any of the following nucleotide sequences:
(1) a partial sequence of the nucleotide sequence of SEQ ID NO:1, or a nucleotide sequence having 80% or more sequence identity to the same;
(2) a complementary sequence to a partial sequence of the nucleotide sequence of SEQ ID NO:1, or a nucleotide sequence having 80% or more sequence identity to the same;
(3) a partial sequence of the nucleotide sequence of SEQ ID NO:2, or a nucleotide sequence having 80% or more sequence identity to the same;
(4) a complementary sequence to a partial sequence of the nucleotide sequence of SEQ ID NO:2, or a nucleotide sequence having 80% or more sequence identity to the same;
(5) a partial sequence of the nucleotide sequence of SEQ ID NO:3, or a nucleotide sequence having 80% or more sequence identity to the same;
(6) a complementary sequence to a partial sequence of the nucleotide sequence of SEQ ID NO:3, or a nucleotide sequence having 80% or more sequence identity to the same;
(7) the nucleotide sequence of SEQ ID NO:4, or a nucleotide sequence having 80% or more sequence identity to the same;
(8) a complementary sequence to the nucleotide sequence of SEQ ID NO:4, or a nucleotide sequence having 80% or more sequence identity to the same;
(9) the nucleotide sequence of SEQ ID NO:6, or a nucleotide sequence having 80% or more sequence identity to the same;
(10) a complementary sequence to the nucleotide sequence of SEQ ID NO:6, or a nucleotide sequence having 80% or more sequence identity to the same;
(11) the nucleotide sequence of SEQ ID NO:8, or a nucleotide sequence having 80% or more sequence identity to the same;
(12) a complementary sequence to the nucleotide sequence of SEQ ID NO:8, or a nucleotide sequence having 80% or more sequence identity to the same;
(13) the nucleotide sequence of SEQ ID NO:10, or a nucleotide sequence having 80% or more sequence identity to the same;
(14) a complementary sequence to the nucleotide sequence of SEQ ID NO:10, or a nucleotide sequence having 80% or more sequence identity to the same;
(15) the nucleotide sequence of SEQ ID NO:12, or a nucleotide sequence having 80% or more sequence identity to the same;
(16) a complementary sequence to the nucleotide sequence of SEQ ID NO:12, or a nucleotide sequence having 80% or more sequence identity to the same;
(17) the nucleotide sequence of SEQ ID NO:13, or a nucleotide sequence having 80% or more sequence identity to the same;
(18) a complementary sequence to the nucleotide sequence of SEQ ID NO:13, or a nucleotide sequence having 80% or more sequence identity to the same;
(19) the nucleotide sequence of SEQ ID NO:14, or a nucleotide sequence having 80% or more sequence identity to the same;
(20) a complementary sequence to the nucleotide sequence of SEQ ID NO:14, or a nucleotide sequence having 80% or more sequence identity to the same;
(21) the nucleotide sequence of SEQ ID NO:15, or a nucleotide sequence having 80% or more sequence identity to the same; and
(22) a complementary sequence to the nucleotide sequence of SEQ ID NO:15, or a nucleotide sequence having 80% or more sequence identity to the same.

9. The method according to any one of claims 1 to 7, wherein the specific oligonucleotide comprises any of the following nucleotide sequences:
(1) a partial sequence of the nucleotide sequence of SEQ ID NO:1, or a nucleotide sequence having 80% or more sequence identity to the same;
(2) a complementary sequence to a partial sequence of the nucleotide sequence of SEQ ID NO:1, or a nucleotide sequence having 80% or more sequence identity to the same;
(3) a partial sequence of the nucleotide sequence of SEQ ID NO:2, or a nucleotide sequence having 80% or more sequence identity to the same;
(4) a complementary sequence to a partial sequence of the nucleotide sequence of SEQ ID NO:2, or a nucleotide sequence having 80% or more sequence identity to the same;
(5) a partial sequence of the nucleotide sequence of SEQ ID NO:3, or a nucleotide sequence having 80% or more sequence identity to the same;
(6) a complementary sequence to a partial sequence of the nucleotide sequence of SEQ ID NO:3, or a nucleotide sequence having 80% or more sequence identity to the same;
(7) the nucleotide sequence of SEQ ID NO:4, or a nucleotide sequence having 80% or more sequence identity to the same;
(8) a complementary sequence to the nucleotide sequence of SEQ ID NO:4, or a nucleotide sequence having 80% or more sequence identity to the same;
(9) the nucleotide sequence of SEQ ID NO:6, or a nucleotide sequence having 80% or more sequence identity to the same;
(10) a complementary sequence to the nucleotide sequence of SEQ ID NO:6, or a nucleotide sequence having 80% or more sequence identity to the same;
(11) the nucleotide sequence of SEQ ID NO:8, or a nucleotide sequence having 80% or more sequence identity to the same;
(12) a complementary sequence to the nucleotide sequence of SEQ ID NO:8, or a nucleotide sequence having 80% or more sequence identity to the same;
(13) the nucleotide sequence of SEQ ID NO:10, or a nucleotide sequence having 80% or more sequence identity to the same;
(14) a complementary sequence to the nucleotide sequence of SEQ ID NO:10, or a nucleotide sequence having 80% or more sequence identity to the same;
(15) the nucleotide sequence of SEQ ID NO:12, or a nucleotide sequence having 80% or more sequence identity to the same;
(16) a complementary sequence to the nucleotide sequence of SEQ ID NO:12, or a nucleotide sequence having 80% or more sequence identity to the same;
(17) the nucleotide sequence of SEQ ID NO:13, or a nucleotide sequence having 80% or more sequence identity to the same;
(18) a complementary sequence to the nucleotide sequence of SEQ ID NO:13, or a nucleotide sequence having 80% or more sequence identity to the same;
(19) the nucleotide sequence of SEQ ID NO:14, or a nucleotide sequence having 80% or more sequence identity to the same;
(20) a complementary sequence to the nucleotide sequence of SEQ ID NO:14, or a nucleotide sequence having 80% or more sequence identity to the same;
(21) the nucleotide sequence of SEQ ID NO:15, or a nucleotide sequence having 80% or more sequence identity to the same; and
(22) a complementary sequence to the nucleotide sequence of SEQ ID NO:15, or a nucleotide sequence having 80% or more sequence identity to the same.

10. The method according to any one of claims 1 to 9, wherein the detection oligonucleotide is a methylated oligonucleotide containing methylated cytosine.

11. The method according to any one of claims 1 to 10, wherein the identification function of the detection oligonucleotide is detection of methylcytosine, detection by a methylated DNA antibody, or detection by a methylcytosine antibody.

12. The method according to any one of claims 1 to 11, wherein the specimen is any of the following specimen:
(a) mammalian blood, body fluid, excreta, body secretion, cell lysate, or tissue lysate;
(b) DNA extracted from one selected from the group consisting of mammalian blood, body fluid, excreta, body secretion, cell lysate, and tissue lysate;
(c) DNA prepared by using as a template RNA extracted from one selected from the group consisting of mammalian tissue, cell, tissue lysate and cell lysate;
(e) DNA extracted from bacterium, fungus or virus; or
(f) DNA prepared by using as a template RNA extracted from bacterium, fungus or virus.

13. The method according to any one of claims 1 to 12, wherein the DNA biological specimen comprising the target DNA region is any of the following DNAs comprising a target DNA region:
(a) DNA digested in advance with a restriction enzyme recognition cleavage site for which is not present in the target DNA region;
(b) DNA comprising a target DNA region and being purified in advance;
(c) free DNA comprising a target DNA region in blood;
(d) DNA comprising a target DNA region and being derived from microbial genome; or
(e) DNA comprising a target DNA region and having been generated from RNA by a reverse transcriptase.

14. The method according to any one of claims 1 to 13, wherein concentration of sodium salt in a solution used in a DNA extracting operation for preparing DNA which is a specimen in First step is 100 mM or more and 1000 mM or less.

15. The method according to any one of claims 1 to 13, wherein concentration of sodium salt in a solution used in a DNA extracting operation for preparing DNA which is a specimen in First step is 100 mM or more and 200 mM or less.

16. A method for selecting a specimen from a cancer patient comprising a step of evaluating that a specimen from a test subject is a specimen from a cancer patient when there is significant difference between a quantification result or a detection result of DNA quantified or detected by using the specimen from the test subject according to the method of any one of claims 1 to 15 and a quantification result or a detection result of DNA quantified or detected by using a specimen from a healthy subject according to the same method, and identifying a specimen from a cancer patient based on a result of the evaluation.

17. The method according to claim 16, wherein the specimen is mammalian serum; and

18. The method according to claim 16 or 17, wherein DNA comprising a target DNA region is free DNA comprising the target DNA region in a mammalian serum.
